# EUROPEAN PATENT APPLICATION

(11) **EP 2 821 491 A1**
(43) Date of publication of application: **07.01.2015**
(21) Application number: 13174901.2
(22) Date of filing: 03.07.2013
(51) Int. Cl.: C12N 15/82

(54) **Method for producing a drought and salt tolerant plant**

(71) Applicant: Rheinische Friedrich-Wilhelms-Universität Bonn, 53113 Bonn (DE)
(72) Inventor: Franke, Rochus, 53757 Sankt Augustin (DE)
(74) Representative: Dick, Alexander

(57) **Abstract**

The present invention is concerned with the improvement of agricobiological traits in plants. More specifically, the present invention pertains to a method for increasing drought-stress and/or salt stress tolerance of a plant comprising expressing in a plant a polynucleotide encoding for a polypeptide having ferulate 5-hydroxylase activity, wherein said polynucleotide is operably linked to a promoter which is active in endodermis cells. The present invention also deals with plants obtainable by the method of the present invention as well as with expression cassettes comprising a polynucleotide encoding for a polypeptide having ferulate 5-hydroxylase activity.

## Description

The present invention is concerned with the improvement of agricobiological traits in plants. More specifically, the present invention pertains to a method for increasing drought-stress and/or salt stress tolerance of a plant comprising expressing in a plant a polynucleotide encoding for a polypeptide having ferulate 5-hydroxylase activity, wherein said polynucleotide is operably linked to a promoter which is active in endodermis cells. The present invention also deals with plants obtainable by the method of the present invention as well as with expression cassettes comprising a polynucleotide encoding for a polypeptide having ferulate 5-hydroxylase activity. Preferably said polynucleotide is operably linked to a promoter which is active in endodermis cells.

Abiotic stresses are serious environmental limitations for the growth of crop plants, reducing the yield of major crop plants by more than 50%. Two of the most limiting abiotic stresses regarding the production of agricultural crops are drought stress and salt stress.

Salinity has a dual effect on plant growth: a) via an osmotic effect on plant water uptake, b) via specific ion toxicities. A decrease of the osmotic potential of the soil solution results in a reduced access of the plant to soil water. If the soil dries, the salt concentration increases, leading to a further decrease of osmotic potential. In order to maintain water uptake from saline soil, plants must osmotically adjust, e.g. by the uptake of salts, or by synthesizing organic solutes.

However, only a few plants can tolerate high salt concentrations in the soil. The most agricultural crop plants only show limited growth, if at all, at high salt concentrations. The main causes for salinization of soil are excessive use of water and inadequate drainage.

Salinity is becoming widespread in many regions, and it there is evidence that serious salinization may affect more than 50 % of all arable crop land worldwide by the year 2050 (see Wang et al, Planta, 2003, 218:1-14).

Moreover, a large proportion of the world's landmass is annually subjected to drought resulting in a major limitation of plant growth. Drought can significantly damage crop plants and, therefore, often adversely affect the yield of said plants.

Although there is technology available that allows to mediate some problems caused by salinity and drougth, e.g. through drainage, irrigation with high quality water, growing plants in the greenhouse, these measures cause high costs.

Thus, breeding of plants that are more stress tolerant has become a promising strategy to mediate some problems that are caused by drought stress and salinity. Plants being more salt tolerant could be grown on land with high levels of salt. Plants being more drought tolerant could be grown in regions affected by drought.

Ferulate 5-hydroxylase (F5H) is a cytochrome P450-dependent monooxygenase that catalyses the hydroxylation of ferulic acid, coniferaldehyde and coniferyl alcohol, leading to sinapic acid and syringyl lignin biosynthesis.

Franke et al. describe the modification of lignin in tobacco and poplar plants over-expressing the Arabidopsis gene encoding ferulate 5-hydroxylase (Franke, R., McMichael, C. M., Meyer, K., Shirley, A. M., Cusumano, J. C. and Chapple, C. (2000), Modified lignin in tobacco and poplar plants over-expressing the Arabidopsis gene encoding ferulate 5-hydroxylase. The Plant Journal, 22: 223-234). However, it is not disclosed that overexpression of this gene allows for improving drought tolerance and/or salt tolerance in plants. Rather, the gene was only linked to the modification of lignin.

Wuys et al. describe that plants over-expressing the Arabidopsis gene encoding ferulate 5-hydroxylase are more resistant against nematodes (Wuyts, N., Lognay, G., Swennen, R., and De Waele, D. (2006). Nematode infection and reproduction in transgenic and mutant Arabidopsis and tobacco with an altered phenylpropanoid metabolism. Journal of Experimental Botany 57, 2825-2835). However, it is not disclosed that overexpression of this gene allows for improving drought tolerance and/or salt tolerance in plants.

There is a need for improving drought tolerance and/or salt tolerance in plants in order to improve agricobiological traits of crop plants.

Accordingly, the technical problem underlying the present invention could seen as the provision of means and methods for complying with the aforementioned needs. The technical problem is solved by the embodiments characterized in the claims and herein below.

Advantageously, it has been found in the context of the studies underlying the present invention that the plants which express a polypeptide having ferulate 5-hydroxylase activity under the control of a promoter also active in the endodermis show increased tolerance to salt and drought stress conditions.

Accordingly, the present invention relates to a method for increasing drought stress and/or salt stress tolerance of a plant comprising expressing in a plant a polynucleotide (preferably, increasing expression of a polynunucleotide) encoding for a polypeptide having ferulate 5-hydroxylase activity.

In particular, the present invention envisages a method for increasing drought stress and/or salt stress tolerance of a plant comprising increasing expression of polynucleotide encoding for a polypeptide having ferulate 5-hydroxylase activity. Preferably, expression of said polynucleotide is increased by introducing and expressing said polynucleotide in the plant.

Preferred methods for introducing a polynucleotide into a plant such as Agrobacterium-mediated transformation are disclosed elsewhere herein.

Preferably, expression of said polynucleotide is increased in endodermis cells.

In a preferred embodiment of the present invention the polynucleotide encoding for a polypeptide having ferulate 5-hydroxylase activity is operably linked to a promoter which is active in endodermis cells.

The plants that are generated by the method of the present invention, show increased tolerance to drought stress and/or salt stress as compared to control plants. Preferably, the increased tolerance to drought stress and/or salt stress is the result of the expression (in particular of the recombinant expression) of the polynucleotide encoding for a polypeptide having ferulate 5-hydroxylase activity.

The term "salt stress" is well understood by the skilled person. As used herein the term, preferably, refers to stress that is induced by an increased concentration of salt. The term "salt" as used herein refers to any water soluble inorganic salt such as sodium sulfate, magnesium sulfate, calcium sulfate, sodium chloride, magnesium chloride, calcium chloride, potassium chloride, etc., salts of agricultural fertilizers and salts associated with alkaline or acid soil conditions.

The term "drought stress" is also well known in the art. As used herein, the term, preferably, refers to stress that is induced by or associated with a deprivation or reduced supply of water.

Preferably, the plant in the context of the present invention has increased tolerance to drought stress and/or salt stress as compared to a control plant. Whether a plant has increased tolerance to salt and/or drought stress can be determined by the skilled person without further ado. Preferably, the increase is statistically significant. Whether an increase is statistically significant can be determined by well known statistical tests including, e.g., Student's t-test, Mann-Whitney test etc.. Accordingly, the plant of the present invention is capable to survive and/or to grow in the presence of increased salt concentrations in the soil (or in any other growth medium) that inhibit growth of a control plant. Further, the plant of the present invention is capable of growing if a reduced supply of water is available.

Furthermore, the plant of the present invention has an increased yield when grown under salt stress and/or drought stress conditions. In particular, it is envisaged that the plant of the present invention has an increased yield as compared to a control plant (grown under the same conditions). Accordingly, the present invention also encompasses a method of increasing yield of a plant under conditions of salt stress and/or drought stress. The method comprises the steps of the aforementioned method of the present invention. In addition, the method may comprise a step of selecting a plant having increased yield under conditions of salt stress and/or drought stress.

The term "yield" as used herein encompasses an increase in biomass (fresh or dry weight) of a plant part or the entire plant, and particularly, harvestable parts of the plant such as the seeds or the fruits. The increase in biomass may be aboveground or underground. Preferably, the increase in yield is statistically significant. More preferably, said increase is an increase of at least 10%, at least 20%, at least 30%, at least 40% or at least 50% in yield.

A control plant is in particular a plant (or a part thereof), or a plant cell which was not produced according to the method of the invention. Preferably, the plant has the same characteristics but does not express the polynucleotide as referred herein (in particular does not express the isolated or recombinant polynucleotide as referred to herein).

The polypeptide as referred to herein in the context of present invention shall have ferulate 5-hydroxylase activity. Preferably, the polypeptide having ferulate 5-hydroxylase activity shall be a cytochrome P450-dependent monooxygenase. Also preferably, the polypeptide shall be a ferulate 5-hydroxylase (abbreviated "F5H"). Thus, it shall be capable of catalyzing the 5-hydroxylation of ferulate (ferulic acid). Also preferably, it shall be capable of catalyzing the 5-hydroxylation of coniferaldehyde and/or coniferyl alcohol.

Polypeptides having F5H activity are well known in the art and are, e.g., described by Weng et al. which is herewith incorporated by reference with respect to its entire disclosure content (Weng, J.K., Li, X., Stout, J., and Chapple, C. (2008). Independent origins of syringyl lignin in vascular plants. Proceedings of the National Academy of Sciences of the United States of America 105, 7887-7892).

Assays for determining, whether a polypeptide has ferulate 5-hydroxylase activity are also well known in the art (see also Weng et al., or Humphreys et al (1999) New routes for lignin biosynthesis defined by biochemical characterization of recombinant ferulate 5-hydroxylase, a multifunctional cytochrome P450-dependent monooxygenase. Proc Natl Acad Sci USA 96:10045-10050).

Further, it is known in the art that polypeptides having F5H activity comprise conserved motifs (see Fig. S1 in Weng et al.). Accordingly, the polypeptide having F5H activity as referred to herein, preferably, comprises at least one of the following motifs: a P450 motif, an N-terminal hydrophobic helix, a proline rich region, a threonine-containing oxygen pocket, and a cysteine-containing heme-binding region. In particular, it is envisaged that it comprises all of the aforementioned motifs. Further, it is envisaged that the polypeptide having F5H activity binds heme.

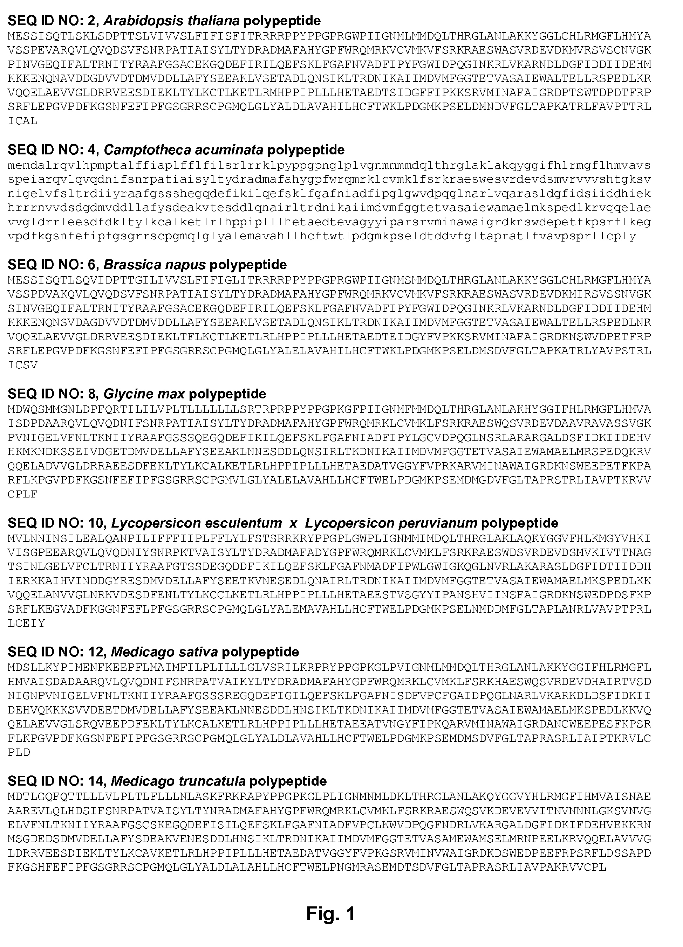

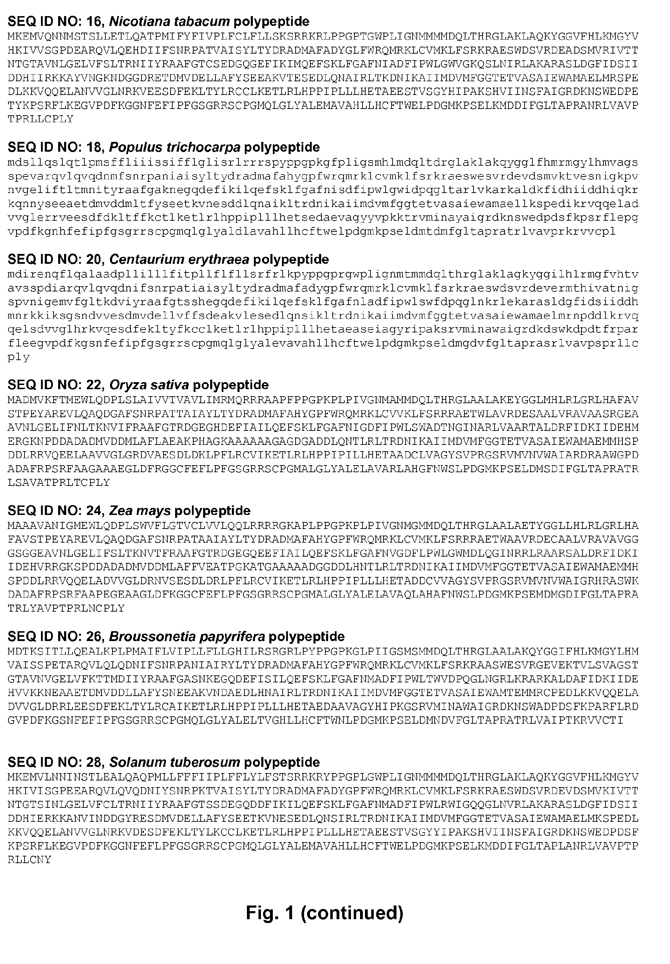

In a preferred embodiment of the present invention, the polynucleotide encoding the polypeptide having F5H activity comprises a nucleic acid selected from the group consisting of
a. a nucleic acid having a nucleotide sequence as shown in SEQ ID No: 1, 3, 5,7,9,11,13,15,17,19,21,23,25, or 27,
b. a nucleic acid encoding a polypeptide having an amino acid sequence as shown in SEQ ID No: 2, 4, 6, 8, 10, 12, 14, 16, 18, 20, 22, 24, 26, or 28, or as shown in Fig. 1;
c. a nucleic acid having a nucleotide sequence being, in increasing order of preference, at least 50%, 60%, 70%, 80%, 90%, or 95% identical to the nucleotide sequence shown in SEQ ID No: 1, 3, 5, 7, 9, 11, 13, 15, 17, 19, 21, 23, 25, or 27, wherein said nucleic acid encodes a polypeptide having ferulate-5-hydroxylase activity;
d. a nucleic acid which hybridizes under stringent conditions to a nucleic acid having a sequence as shown in SEQ ID No: 1, 3, 5, 7, 9, 11, 13, 15, 17, 19, 21, 23, 25, or 27, wherein said nucleic acid encodes a polypeptide having ferulate-5-hydroxylase activity, and
e. a nucleic acid encoding a polypeptide having an amino acid sequence being in increasing order of preference, at least 50%, 60%, 70%, 80%, 90% or 95% identical to the amino acid sequence shown in SEQ ID No: 2, 4, 6, 8, 10, 12, 14, 16, 18, 20, 22, 24, 26, or 28, wherein said polypeptide has ferulate-5-hydroxylase activity.

The term "polynucleotide" as used herein refers to a linear or circular nucleic acid molecule. It encompasses DNA as well as RNA molecules. The polynucleotide of the present invention shall be provided, preferably, either as an isolated polynucleotide (i.e. isolated from its natural context) or in genetically modified form. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides. The polynucleotide of the present invention is **characterized in that** it shall encode a polypeptide as referred to above. The polynucleotide, preferably, has a specific nucleotide sequence as mentioned above. Moreover, due to the degeneracy of the genetic code, polynucleotides are encompassed which encode a specific amino acid sequence as recited above.

Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence **characterized in that** the sequence can be derived from the aforementioned specific nucleic acid sequences by at least one nucleotide substitution, addition and/or deletion whereby the variant nucleic acid sequence shall still encode a polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6x sodium chloride/sodium citrate (= SSC) at approximately 45°C, followed by one or more wash steps in 0.2 x SSC, 0.1 % SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 x SSC (pH 7.2). If organic solvent is present in the abovementioned buffer, for example 50% formamide, the temperature under standard conditions is approximately 42°C. The hybridization conditions for DNA:DNA hybrids are preferably for example 0.1 x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford.

Preferred stringent hybridization conditions are disclosed elsewhere herein. Preferably, the stringent conditions comprise hybridisation in 4xSSC at 65°C or in 4xSSC and 50% formamide at 45°C, and washing in 1xSSC and 65°C. More preferably, the stringent conditions comprise hybridisation in 1xSSC at 65°C or in 1xSSC and 50% formamide at 42°C, and washing in 0.3xSSC and 65°C.

Alternatively, polynucleotide variants are obtainable by PCR-based techniques such as mixed oligonucleotide primer- based amplification of DNA, i.e. using degenerated primers against conserved domains of the polypeptides of the present invention. Conserved domains of the polypeptide of the present invention may be identified by a sequence comparison of the nucleic acid sequence of the polynucleotide or the amino acid sequence of the polypeptide of the present invention with sequences of other members of the enzyme families referred to in accordance with this invention. Oligonucleotides suitable as PCR primers as well as suitable PCR conditions are described in the accompanying Examples. As a template, DNA or cDNA from bacteria, fungi, plants or animals may be used. Further, variants include polynucleotides comprising nucleic acid sequences which are at least 60% or at at 70%, at least 75%, at least 80%, at least 85%, at least 90%, at least 95%, at least 98% or at least 99% identical to the specific nucleic acid sequences. Moreover, also encompassed are polynucleotides which comprise nucleic acid sequences encoding amino acid sequences which are at least 60 %, at least 70%, at least 75%, at least 80%, at least 85%, at least 90%, at least 95%, at least 98% or at least 99% identical to the specific amino acid sequences referred to herein.

The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp ( Higgins 1989, CABIOS, 5 1989: 151-153) or the programs Gap and BestFit (Needleman 1970, J. Mol. Biol. 48; 443-453 and Smith 198, Adv. Appl. Math. 2; 482-489), which are part of the GCG software packet from Genetics Computer Group, 575 Science Drive, Madison, Wisconsin, USA 53711, version 1991, are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the aforementioned nucleic acid sequences is also encompassed as a polynucleotide of the present invention. The fragment shall encode a polypeptide which still has the activity as specified above. Accordingly, the polypeptide may comprise or consist of the domains of the polypeptide of the present invention conferring the said biological activity. A fragment as meant herein, preferably, comprises at least 50, at least 100, at least 250 or at least 500 consecutive nucleotides of any one of the aforementioned nucleic acid sequences or encodes an amino acid sequence comprising at least 20, at least 30, at least 50, at least 80, at least 100 or at least 150 consecutive amino acids of any one of the aforementioned amino acid sequences.

Preferably, the polynucleotide encoding for a polypeptide having F5H activity referred to above are expressed from heterologous polynucleotides, i.e. from polynucleotides which have been either transiently, e.g., by using an expression vector, or stably, e.g., by using Tor insertion, introduced into the plant cell. The term "heterologous" as used herein means that the polynucleotide does not occur naturally in the plant cell (and, thus, shall be a transgene). The term, thus, encompasses modified or unmodified polynucleotides which are derived from different organisms or modified polynucleotides derived from the plant cell of the invention. It is to be understood that the heterologous polynucleotide comprise a promoter which allow for expression in endodermis cells as set forth elsewhere, thereby forming an expression cassette.

Preferably, the polynucleotide encoding for a polypeptide having ferulate 5-hydroxylase activity shall be operably linked to a promoter which is active in endodermis cells.

The term "promoter" as used herein refers to a nucleic acid which is capable of governing the expression of a polynucleotide operatively linked thereto, i.e. a polynucleotide encoding for a polypeptide having ferulate 5-hydroxylase activity referred to elsewhere in this specification in detail. A promoter as referred to in accordance with the present invention, preferably, comprises sequence motifs which are recognized and bound by polypeptides, i.e. transcription factors. Thereby the expression of a nucleic acid operatively linked to the expression control sequence will be initiated. Preferably, the promoter as used herein is derived from a plant.

The term "operably linked" as used herein is intended to mean that the polynucleotide encoding for a polypeptide having ferulate 5-hydroxylase activity is linked to the promoter in a manner that allows for expression of said polynucleotide in endodermis cells.

The term "endodermis" is well known by the skilled person. As used herein, the term, preferably refers to central, innermost layer of root cortex. It is made of compact living cells that are impregnated with hydrophobic substances to restrict apoplastic flow of water to the inside and also the backflow to the outside. The endodermis is the boundary between the cortex and the stele. The endodermis limits water, and any solutes dissolved in the water, to pass through this layer via the apoplast pathway.

An endodermis cell is preferably a cell which is comprised by the endodermis of a plant. The cells of the endodermis have their primary cell walls modified on four sides radial and transverse with suberin, a water permeability reducing lipophilic substance.

Preferably, the endodermis cells comprise suberin. The term "suberin" as used herein, preferably refers to a plant-specific cell wall-associated hydrophobic polymer containing a fatty acid-derived domain and an aromatic domain.

The promoter to be used in the context of the present invention, preferably, shall be active in endodermis cells. Whether a promoter is active in endodermis cells can be determined by the skilled person with out further ado. For example, the promoter may be operatively linked to a reporter gene such as a luciferase or a beta-glucuronidase (GUS) gene. In principle, a promoter is active in endodermis cells, if reporter gene activity can be detected in endodermis cells.

In an embodiment of the present invention, the promoter which is active in endodermis cells is a endodermis-specific or endodermis-preferential promoter. Accordingly, the promoter shall be capable of specifically or preferentially initiating transcription in endodermis cells. Thus, the promoter shall be predominantly active in endodermis cells. A preferred endodermis-specific promoter are a promoter of a cytochrome P450 CYP86A1 gene, or of an orthologue thereof, and a promoter of a cytochrome CYP86B1 gene, or of an orthologue thereof. Preferred sequences are disclosed herein below.

A endodermis preferential promoter as used herein, preferably, is a promoter refers to a promoter which is has greater activity in endodermis cells than in the majority of other cell types of the plant.

Also preferably, the promoter which is active in endodermis cells is a endodermis-specific or endodermic-preferential promoter is a vascular bundle specific or preferential promoter.

It is to be understood, however, that the invention is not limited to endodermis-specific or endodermic-preferential promoters, or to vascular bundle specific or preferential promoters.

In an embodiment, the promoter which is active in endodermis cells is not the CaMV 35S promoter.

Preferably, the promoter which is active in endodermis cells is heterologous with respect to the polynucleotide encoding for a polypeptide having ferulate-5-hydroxylase activity. Accordingly, the promoter which allows expression of the polynucleotide encoding a polypeptide having ferulate-5-hydroxylase activity in endodermis cells shall not be the native promoter of said polynucleotide.

In a preferred embodiment of the present invention, the promoter which is active in endodermis cells is selected from the group consisting of a promoter of a cinnamate 4-hydrolylase gene, a promoter ofp-coumaroyl shikimate 3 hydroxlase gene, a promoter of a cytochrome P450 CYP86A1 gene (a gene which encodes for a fatty acid omega hydroxylase), or of an orthologue thereof, and a promoter of a cytochrome CYP86B1 gene (a gene which encodes for a fatty acid omega hydroxylase) or of an orthologue thereof.

Preferably, said promoters are the promoters which naturally drive the expression of said genes.

Preferably, the promoter to be used in the context of the present invention comprises a region of at least 300 bp, more preferably, of at least 500 bp and, most preferably, of at least 1000 bp upstream of the transcription start site or of the start codon ATG of the aforementioned genes.

It is, in particular, envisaged that the promoter of of a cinnamate 4-hydrolylase gene or a promoter of p-coumaroyl shikimate 3 hydroxlase gene is used.

Also preferably, the aforementioned genes are plant genes. More preferably, said genes are derived from Arabidopsis thaliana. Most preferably, the cinnamate 4-hydrolylase gene has the Arabidopsis code At2g30490, the p-coumaroyl shikimate 3 hydroxlase gene has the Arabidopsis code At2g40890, the cytochrome P450 CYP86A1 gene has the Arabidopsis code At5g58860, the cytochrome P450 CYP86B1 gene has the Arabidopsis code At5g23190.

Orthologues of the CYP86A1 gene and the CYP86B1 genes are well known in the art. For example, the rice orthologue of the CYP86A1 is CYP86A9, and the rice orthologue of CYP86B1 is CYP86B3.

A promoter of a cinnamate 4-hydrolylase gene (as well as the corresponding gene) is disclosed by Bell-Lelong et al. (Bell-Lelong, D.A., Cusumano, J.C., Meyer, K., and Chapple, C. (1997). Cinnamate-4-hydroxylase expression in Arabidopsis - Regulation in response to development and the environment. Plant Physiology 113, 729-738). A promoter of p-coumaroyl shikimate 3 hydroxlase gene (as well as the corresponding gene) is disclosed in Nair et al. (Nair, R.B., Xia, Q., Kartha, C.J., Kurylo, E., Hirji, R.N., Datla, R., and Selvaraj, G. (2002). Arabidopsis CYP98A3 mediating aromatic 3-hydroxylation. Developmental regulation of the gene, and expression in yeast. Plant Physiology 130, 210-220). This paper also discloses the C4H promoter as well as its activity in the endodermis. A promoter of a cytochrome P450 CYP86A1 gene (as well as the corresponding gene) is disclosed by Höfer et al. (Höfer , R., Briesen, I., Beck, M., Pinot, F., Schreiber, L., and Franke, R. (2008). The Arabidopsis cytochrome P450 CYP86A1 encodes a fatty acid ω-hydroxylase involved in suberin monomer biosynthesis. Journal of Experimental Botany 59, 2347-2360). A promoter of a cytochrome CYP86B1 gene (as well as the corresponding gene) is disclosed by Compagnon et al. (Compagnon, V., Diehl, P., Benveniste, I., Meyer, D., Schaller, H., Schreiber, L., Franke, R., and Pinot, F. (2009). CYP86B1 is required for very long chain ω-hydroxyacids and α,ω-dicarboxylic acids synthesis in root and seed suberin polyester. Plant Physiology 150, 1831-1843).

In a preferred embodiment of the present invention, the promoter which is active in endodermis cells comprises a nucleic acid selected from the group consisting of:
a. a nucleic acid having a nucleotide sequence as shown in SEQ ID No: 29, 30, 31, 32 or 33;
b. a nucleic acid having a nucleotide sequence being at least 50% identical to the nucleotide sequence shown in SEQ ID No: 29, 30, 31, 32 or 33,
c. a nucleic acid which hybridizes under stringent conditions to a nucleic acid having a sequence as shown in SEQ ID No: 29, 30, 31, 32 or 33, and
d. a nucleic acid being a fragment of any one of the nucleic acids as set forth under a, b and c.

SEQ ID NO: 29 is the sequence of the Arabidopsis thaliana cinnamate 4-hydroxylase promoter. The last three nucleic acids represent the start codon. SEQ ID NO: 33 is the sequence of the Arabidopsis thaliana cinnamate 4-hydroxylase promoter as used in the studies underlying the present invention.

SEQ ID NO: 30 is the sequence of the Arabidopsis thaliana p-coumaroyl shikimate 3'-hydroxylase promoter. The last three nucleic acids represent the start codon.

SEQ ID NO: 31 is the sequence of the Arabidopsis thaliana CYP86B1 promoter. The last three nucleic acids represent the start codon.

SEQ ID NO: 32 is the sequence of the Arabidopsis thaliana CYP86A1 promoter. The last three nucleic acids represent the start codon.

Of course, the promoter having a sequence as set forth under b) to d) shall be active in endodermis cells. In particular, it is envisaged that nucleic acids have substantially the same activity, preferably in the endodermis as the promoter having a nucleotide sequence as shown in SEQ ID No: 29, 30, 31, 32 and 33, respectively.

Preferably, the nucleic acids under b) include polynucleotides comprising, in increasing order of preference, nucleic acid sequences which are at least 60% or at at 70%, at least 75%, at least 80%, at least 85%, at least 90%, at least 95%, at least 98% or at least 99% identical to the specific nucleic acid sequences.

The fragment referred to under d), preferably, has a length of at least 300 bp, more preferably, of at least 500 bp and, most preferably, or of at least 1000 bp. Preferably, the fragment comprises the 3' end of the aforementioned sequenced. Preferred fragments comprises at least 500 bp or 1000 bp of the 3' end of the nucleotide sequence shown in SEQ ID No: 29, 30, 31, 32 or 33.

Preferred hybridization conditions are disclosed elsewhere herein.

Further preferred promoters which are active in endodermis cells are the promoters of the lipid transfer protein (LTP8, At2g18370), of glycerol-3-phosphate acyltransferase GPAT5 (At3gll430), fatty acyl-CoA reductases FAR1 (At5g22500) FAR4 (At3g44540) and FAR5 (At3g44550), Casparian strip membrane domain protein CASP1 (At2g36100) and aliphatic suberin feruloyl transferase ASFT (At5g41040), see also Naseer et al. PNAS June 19, 2012 vol. 109 no. 25 10101-10106).

Preferably, the polynucleotide or expression cassette as referred to herein is introduced and expressed in a plant. Thereby, transgenic plants are generated. Accordingly, the polynucleotide or the expression cassette as set forth herein, shall be present as a transgene. Preferably, transgenic plants can be obtained by Agrobacterium-mediated transformation. Accordingly, the polynucleotide as set forth herein, is preferably, introduced into plants by Agrobacterium-mediated transformation. However, other methods for introducing transgenes are envisaged as well, such as biolistic bombardment.

In an embodiment of the present invention, the method comprises the step of selecting a plant having increased drought-stress and/salt stress tolerance.

The present invention is also directed to an expression cassette comprising a polynucleotide encoding for a polypeptide having ferulate 5-hydroxylase activity, wherein said polynucleotide is operably linked to a promoter which is active in endodermis cells.

The present invention also relates to a vector comprising the expression cassette or the polynucleotide of the present invention.

The term "vector", preferably, encompasses plasmids, expression vectors, T-DNA vectors as well as artificial chromosomes, such as bacterial or yeast artificial chromosomes. The vector encompassing the polynucleotides of the present invention, preferably, further comprises selectable markers for propagation and/or selection in a host. The vector may be introduced into a host cell by various techniques well known in the art. If introduced into a host cell, the vector, or the T-DNA region of the vector comprising the polynucleotide as set forth herein be incorporated into the genome. can be introduced into prokaryotic or eukaryotic cells via conventional transformation or transfection techniques.

Preferably, the vector referred to herein is suitable as a cloning vector, i.e. replicable in microbial systems. Such vectors ensure efficient cloning in bacteria and, preferably, yeasts or fungi and make possible the stable transformation of plants. "Cloning vectors" typically contain restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide, e.g., kanamycin resistance, streptomycin resistance, spectinomycin resistance, tetracycline resistance, hygromycin resistance or ampicillin resistance.

Those vector systems which must be mentioned are, in particular, various binary and co-integrated vector systems which are suitable for the T DNA-mediated transformation. Such vector systems are, as a rule, **characterized in that** they contain at least the vir genes, which are required for the Agrobacterium-mediated transformation, and the sequences which delimit the T-DNA (T-DNA border). A particular preferred vector is a T-DNA vector comprising a left and right border region for introducing a T-DNA comprising the polynucleotide as referred to herein into a host cell.

The present invention also contemplates a plant or a host cell comprising a polynucleotide encoding for a polypeptide having ferulate 5-hydroxylase activity, wherein said polynucleotide is operably linked to a promoter which is active in endodermis cells, the expression cassette or the vector of the present invention. Also envisaged is plant obtainable by the method of the present invention. Preferably, said plant comprises, said polynucleotide, said expression cassette or said vector.

Also envisaged by the present invention is seed comprising a polynucleotide encoding for a polypeptide having ferulate 5-hydroxylase activity, wherein said polynucleotide is operably linked to a promoter which is active in endodermis cells, the expression cassette or the vector of the present invention.

Preferably, said polynucleotide is present in the plant, seed or host cell as a transgene. Also preferably, it should be stably integrated into the genome of the plant.

The term "plant" as used herein encompasses whole plants, ancestors and progeny of the plants and plant parts, including seeds, shoots, stems, leaves, roots (including tubers), fruits, stalk, seedlings, tubers, flowers, and tissues and organs, wherein each of the aforementioned comprise a polynucleotide encoding for a polypeptide having ferulate 5-hydroxylase activity, wherein said polynucleotide is operably linked to a promoter which is active in endodermis cells. The term "plant" also encompasses plant cells, suspension cultures, callus tissue, embryos, meristematic regions, pollen, and microspores, again wherein each of the aforementioned comprise the said polynucleotide. Preferably, the plant is a vascular plant.

Preferred plants are those plants belonging to the genera Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solarium, Petunia, Digitalis, Majorana, Cichorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea and Populus.

Preferably, the plant is a dicotyledonous or a monocotyledonous plant.

A "monocotyledonous plant" as used herein, preferably, refers to a flowering plant with one cotyledon in the seed. Particularly preferred monocotyledonous plants (herein also referred to as monocots) are maize, wheat, rice, barley, oat, rye, sorghum, millet, banana, ryegrass or coix. The term "monocotyledonous plant" includes, preferably, plants of the genera of the subfamilies Andropogonoideae (particularly, the genera Saccharum, Sorghum, or Zea), Arundineae (particularly, the genus Phragmites), Oryzoideae (particularly, the genus Oryza), Panicoideae, and, more preferably, Pooideae (Festuciadeae) (particularly, the genera Poa, Festuca, Lolium, Trisetum, Agrostis, Phleum, Dactylis, Alopecurus, Avena, Triticum, Secale, and Hordeum). Preferred mon-ocotyledonous plants are Avena sativa (oats), Saccharum officinarum (sugarcane), Triticum dicoccum (Emmer wheat), Triticum monococcum (Einkorn wheat), Triticum spelta (spelt wheat), Triticum durum (wheat), Triticum turgidum, Triticum aestivum (wheat), Zea mays (maize/corn), Panicum miliaceum (common millet), Pennisetum thiphoides (Bulrush millet), Hordeum vulgare or H. sativum (barley), Oryza sativa (rice), Zizania aquatica (wild rice), Secale cereale (rye), Sorghum bicolor (S. vulgare) (sorghum). More preferred are wheat (Triticum spp.), rice (Oryza spp.), barley (Hordeum spp.), oats (Avena spp.), rye (Secale spp.), corn (Zea mays), sorghum and millet (Pennisettum spp).

A "dicotyledonous plant" refers to a plant whose seeds have two cotyledons. Preferred dicotyledonous plants are selected in particular from the dicotyledonous crop plants for example, Asteraceae such as sunflower, tagetes or calendula and others, Compositae, Cruciferae, particularly the genus Brassica, in particular specis napus (oilseed rape), campestris (beet), oleracea; the genus Arabidopsis , in particular species thaliana, Cucurbitaceae, Leguminosae, particularly the genus Glycine, in particular the species max (soybean), soya, and alfalfa, pea, beans or peanut and others, Solanaceae, in particular the genus Lycopersicon , very particularly the species esculentum (tomato), the genus Solanum, very particularly the species tuberosum, and linseed, cotton, hemp, flax, cucumber, spinach, carrot, sugar beet and the various tree, nut and grapevine species, in particular banana and kiwi fruit.

In an embodiment of the present invention, the plant is Arabidopsis thaliana. However, it is also envisaged that the term "plant" does not encompass Arabidopsis thaliana plants or plants belonging to the genus Arabidopsis. In an embodiment of the present invention, the term "plant" does not encompass plants belonging to the genus populous, and, thus poplar plants.

In a preferred embodiment, the plant is a crop plant. Preferred crop plants are rice, soybean, wheat, barley, maize, and potato and cotton.

Host cells are, preferably, transgenic cells or cell lines derived from plants, in particular from the plants as referred to above. More preferably, said host cells are derived from as set forth herein above. The host cells derived from plants encompass cells of certain tissues, organs and parts of plants in all their phenotypic forms such as anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. Preferably, the term "host cell" encompasses root tissue. In particular, the term "host cell" encompasses endodermis cells, i.e. endodermis cells derived from a plant.

It is to be understood that the polynucleotide or vector according to the present invention may also be present in prokaryotic or eukaryotic single cell organism (also referred to as micro-organisms), particularly for cloning purpose (for example, in E. coli), and for plant transformation (for example, in Agrobacterium). Thus, the term "host cell", preferably, also encompasses prokaryotic or eukaryotic single cell organisms (also referred to as micro-organisms). Particularly contemplated as prokaryotic host cells in the context of the present invention are Rhizobiaceae cells, in particular of the genus Agrobacterium. Preferred Agrobacterium cells are Agrobacterium tumefaciens and Agrobacterium rhizogenes cells.

Finally, the present invention relates to the use of a polynucleotide encoding for a polypeptide having ferulate 5-hydroxylase activity, said polynucleotide being operably linked to a promoter which is active in endodermis cells, of the expression cassette of the present invention, or of the vector of the present invention for i) increasing drought-stress and/or salt stress tolerance of a plant and/or for ii) improving yield of a plant grown under conditions of drought and/or salt stress.

The figures show:
Fig. 1: Amino acid sequences of preferred ferulate 5-hydroxylases
Fig. 2: Wild-type and C4H-F5H transgenic plants cultivated under drought stress conditions (Top left: wild-type, watered, Top right: wild-type without irrigation; bottom: C4H-F5H without irrigation).

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Example 1

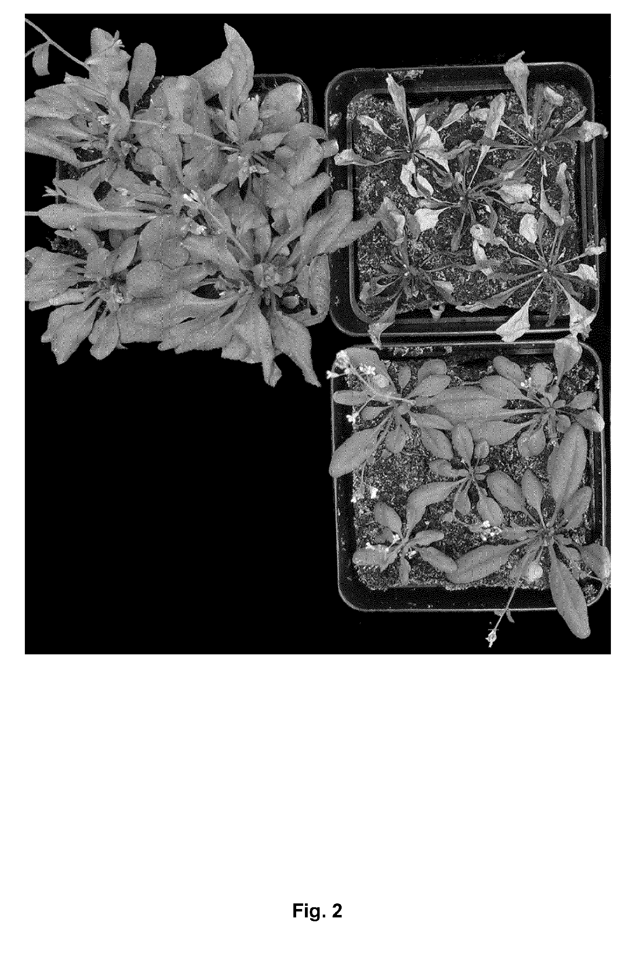

Drought stress experiment:
C4H-F5H transgenic Arabidopsis plants (comprising a construct encoding for a ferulate 5-hydroxylase (SEQ ID NO: 2) under control of the cinnamate 4-hydrolylase promoter from Arabidopsis thaliana) were cultivated in Floradur potting mix (Floraguard, Oldenburg,
Germany) at a light intensity of 100 µE m⁻² see⁻¹ at 22°C under a photoperiod of 16-h light/8-h dark. Plants were irrigated three times a week. Three weeks after sawing irrigation was stopped and plants were cultivated for additional 10-12 days and inspected for symptoms. Wild type plants were extremely wilted and could not be regrown/recovered after rewatering. C4H-F5H transgenic plants were still green and turgescent and could be further cultivated by rewatering (see Fig. 2).

## Claims

1. A method for increasing drought-stress and/or salt stress tolerance of a plant comprising expressing, in particular introducing and expressing, in a plant a polynucleotide encoding for a polypeptide having ferulate 5-hydroxylase activity.

2. The method of claim 1, wherein the polypeptide having ferulate 5-hydroxylase activity is capable of catalyzing the hydroxylation of ferulic acid.

3. The method for method according to claims 1 and 2, wherein the polynucleotide encoding for a polypeptide having ferulate 5-hydroxylase activity comprises a nucleic acid selected from the group consisting of
a. a nucleic acid having a nucleotide sequence as shown in SEQ ID No: 1, 3, 5, 7, 9, 11, 13, 15, 17, 19, 21, 23, 25, or 27;
b. a nucleic acid encoding a polypeptide having an amino acid sequence as shown in SEQ ID No: 2, 4, 6, 8, 10, 12, 14, 16, 18, 20, 22, 24, 26, or 28;
c. a nucleic acid having a nucleotide sequence being at least 50% identical to the nucleotide sequence shown in SEQ ID No: 1, 3, 5, 7, 9, 11, 13, 15, 17, 19, 21, 23, 25, or 27, wherein said nucleic acid encodes a polypeptide having ferulate-5-hydroxylase activity;
d. a nucleic acid which hybridizes under stringent conditions to a nucleic acid having a sequence as shown in SEQ ID No: 1, 3, 5, 7, 9, 11, 13, 15, 17, 19, 21, 23, 25, or 27, wherein said nucleic acid encodes a polypeptide having ferulate-5-hydroxylase activity, and
e. a nucleic acid encoding a polypeptide having an amino acid sequence being at least 50% identical to the amino acid sequence shown in SEQ ID No: 2, 4, 6, 8, 10, 12, 14, 16, 18, 20, 22, 24, 26, or 28, wherein said polypeptide has ferulate-5-hydroxylase activity.

4. The method of any one of claims 1 to 3, wherein the wherein said polynucleotide is operably linked to a promoter which is active in endodermis cells.

5. The method of claim 4, wherein the promoter is heterologous with respect to the polynucleotide encoding polypeptide having ferulate-5-hydroxylase activity.

6. The method according to any one of claims 1 to 5, wherein the promoter is selected from the group consisting of a promoter of a cinnamate 4-hydrolylase gene, a promoter of p-coumaroyl shikimate 3 hydroxlase gene, a promoter of a cytochrome P450 CYP86A1 gene, and a promoter of a cytochrome CYP86B1 gene.

7. The method according to claims 1 to 6, wherein the plant is a dicotyledonous or a monocotyledonous plant.

8. The method according to any one of claims 1 to 6, wherein the plant is a crop plant, in particular rice, wheat, barley, maize or cotton.

9. A plant obtainable by the method according to any one of claims 1 to 8, wherein the plant comprises the polynucleotide encoding for a polypeptide having ferulate 5-hydroxylase activity, wherein said polynucleotide encoding for said polypeptide having ferulate 5-hydroxylase activity is operably linked to a promoter which is active in endodermis cells.

10. The plant according to claims 9, wherein the plant is not Arabidopsis thaliana.

11. An expression cassette comprising a polynucleotide encoding for a polypeptide having ferulate 5-hydroxylase activity, wherein said polynucleotide is operably linked to a heterologous promoter, in particular to a promoter which is active in endodermis cells, and wherein said polynucleotide comprises a nucleic acid selected from the group consisting of
a. a nucleic acid having a nucleotide sequence as shown in SEQ ID No: 1, 3, 5, 7, 9, 11, 13, 15, 17, 19, 21, 23, 25, or 27;
b. a nucleic acid encoding a polypeptide having an amino acid sequence as shown in SEQ ID No: 2, 4, 6, 8, 10, 12, 14, 16, 18, 20, 22, 24, 26, or 28;
c. a nucleic acid having a nucleotide sequence being at least 50% identical to the nucleotide sequence shown in SEQ ID No: 1, 3, 5, 7, 9, 11, 13, 15, 17, 19, 21, 23, 25, or 27, wherein said nucleic acid encodes a polypeptide having ferulate-5-hydroxylase activity;
d. a nucleic acid which hybridizes under stringent conditions to a nucleic acid having a sequence as shown in SEQ ID No: 1, 3, 5, 7, 9, 11, 13, 15, 17, 19, 21, 23, 25, or 27, wherein said nucleic acid encodes a polypeptide having ferulate-5-hydroxylase activity, and
e. a polynucleotide encoding a polypeptide having an amino acid sequence being at least 50% identical to the amino acid sequence shown in SEQ ID No: 2, 4, 6, 8, 10, 12, 14, 16, 18, 20, 22, 24, 26, or 28, wherein said polypeptide has ferulate-5-hydroxylase activity.

12. The expression cassette of claim 11, wherein the promoter is selected from the group consisting of a promoter of a cinnamate 4-hydrolylase gene, a promoter of p-coumaroyl shikimate 3 hydroxlase gene, a promoter of a cytochrome P450 CYP86A1 gene, and a promoter of a cytochrome CYP86B1 gene.

13. A vector comprising the expression cassette of claims 11 and 12.

14. A plant or a plant cell, comprising the expression cassette of claims 11 and 12, or the vector of claim 13.

15. Use of a polynucleotide encoding for a polypeptide having ferulate 5-hydroxylase activity, of the expression cassette of claims 11 and 12, or of the vector of claim 13 for increasing drought-stress and/or salt stress tolerance of a plant, and/or for improving yield of a plant grown under conditions of drought and/or salt stress.


SEQUENCE LISTING

<110>  Rheinische Friedrich-Wilhelms-Universität Bonn

<120>  Method for producing drought and salt tolerant plants

<130>  P11862EP

<160>  33

<170>  PatentIn version 3.5

<210>  1
<211>  1563
<212>  DNA
<213>  Arabidopsis thaliana

<400>  1
atggagtctt ctatatcaca aacactaagc aaactatcag atcccacgac gtctcttgtc      60

atcgttgtct ctcttttcat cttcatcagc ttcatcacac ggcggcgaag gcctccatat     120

cctcccggtc cacgaggttg gcccatcata ggcaacatgt taatgatgga ccaactcacc     180

caccgtggtt tagccaattt agctaaaaag tatggcggat tgtgccatct ccgcatggga     240

ttcctccata tgtacgctgt ctcatcaccc gaggtggctc gacaagtcct tcaagtccaa     300

gacagcgtct tctcgaaccg gcctgcaact atagctataa gctatctgac ttacgaccga     360

gcggacatgg ctttcgctca ctacggaccg ttttggagac agatgagaaa agtgtgtgtc     420

atgaaggtgt ttagccgtaa aagagctgag tcatgggctt cagttcgtga tgaagtggac     480

aaaatggtcc ggtcggtctc ttgtaacgtt ggtaagccta taaacgtcgg ggagcaaatt     540

tttgcactga cccgcaacat aacttaccgg gcagcgtttg ggtcagcctg cgagaaggga     600

caagacgagt tcataagaat cttacaagag ttctctaagc tttttggagc cttcaacgta     660

gcggatttca taccatattt cgggtggatc gatccgcaag ggataaacaa gcggctcgtg     720

aaggcccgta atgatctaga cggatttatt gacgatatta tcgatgaaca tatgaagaag     780

aaggagaatc aaaacgctgt ggatgatggg gatgttgtcg ataccgatat ggttgatgat     840

cttcttgctt tttacagtga agaggccaaa ttagtcagtg agacagcgga tcttcaaaat     900

tccatcaaac ttacccgtga caatatcaaa gcaatcatca tggacgttat gtttggagga     960

acggaaacgg tagcgtcggc gatagagtgg gccttaacgg agttattacg gagccccgag    1020

gatctaaaac gggtccaaca agaactcgcc gaagtcgttg gacttgacag acgagttgaa    1080

gaatccgaca tcgagaagtt gacttatctc aaatgcacac tcaaagaaac cctaaggatg    1140

cacccaccga tccctctcct cctccacgaa accgcggagg acactagtat cgacggtttc    1200

ttcattccca agaaatctcg tgtgatgatc aacgcgtttg ccataggacg cgacccaacc    1260

tcttggactg acccggacac gtttagacca tcgaggtttt tggaaccggg cgtaccggat    1320

ttcaaaggga gcaatttcga gtttataccg ttcgggtcgg gtcgtagatc gtgcccgggt    1380

atgcaactag ggttatacgc gcttgactta gccgtggctc atatattaca ttgcttcacg     1440

tggaaattac ctgatgggat gaaaccaagt gagctcgaca tgaatgatgt gtttggtctc     1500

acggctccta aagccacgcg gcttttcgcc gtgccaacca cgcgcctcat ctgtgctctt     1560

taa                                                                   1563

<210>  2
<211>  520
<212>  PRT
<213>  Arabidopsis thaliana

<400>  2

Met Glu Ser Ser Ile Ser Gln Thr Leu Ser Lys Leu Ser Asp Pro Thr
1               5                   10                  15

Thr Ser Leu Val Ile Val Val Ser Leu Phe Ile Phe Ile Ser Phe Ile
            20                  25                  30

Thr Arg Arg Arg Arg Pro Pro Tyr Pro Pro Gly Pro Arg Gly Trp Pro
        35                  40                  45

Ile Ile Gly Asn Met Leu Met Met Asp Gln Leu Thr His Arg Gly Leu
    50                  55                  60

Ala Asn Leu Ala Lys Lys Tyr Gly Gly Leu Cys His Leu Arg Met Gly
65                  70                  75                  80

Phe Leu His Met Tyr Ala Val Ser Ser Pro Glu Val Ala Arg Gln Val
                85                  90                  95

Leu Gln Val Gln Asp Ser Val Phe Ser Asn Arg Pro Ala Thr Ile Ala
            100                 105                 110

Ile Ser Tyr Leu Thr Tyr Asp Arg Ala Asp Met Ala Phe Ala His Tyr
        115                 120                 125

Gly Pro Phe Trp Arg Gln Met Arg Lys Val Cys Val Met Lys Val Phe
    130                 135                 140

Ser Arg Lys Arg Ala Glu Ser Trp Ala Ser Val Arg Asp Glu Val Asp
145                 150                 155                 160

Lys Met Val Arg Ser Val Ser Cys Asn Val Gly Lys Pro Ile Asn Val
                165                 170                 175

Gly Glu Gln Ile Phe Ala Leu Thr Arg Asn Ile Thr Tyr Arg Ala Ala
            180                 185                 190

Phe Gly Ser Ala Cys Glu Lys Gly Gln Asp Glu Phe Ile Arg Ile Leu
        195                 200                 205

Gln Glu Phe Ser Lys Leu Phe Gly Ala Phe Asn Val Ala Asp Phe Ile
    210                 215                 220

Pro Tyr Phe Gly Trp Ile Asp Pro Gln Gly Ile Asn Lys Arg Leu Val
225                 230                 235                 240

Lys Ala Arg Asn Asp Leu Asp Gly Phe Ile Asp Asp Ile Ile Asp Glu
                245                 250                 255

His Met Lys Lys Lys Glu Asn Gln Asn Ala Val Asp Asp Gly Asp Val
            260                 265                 270

Val Asp Thr Asp Met Val Asp Asp Leu Leu Ala Phe Tyr Ser Glu Glu
        275                 280                 285

Ala Lys Leu Val Ser Glu Thr Ala Asp Leu Gln Asn Ser Ile Lys Leu
    290                 295                 300

Thr Arg Asp Asn Ile Lys Ala Ile Ile Met Asp Val Met Phe Gly Gly
305                 310                 315                 320

Thr Glu Thr Val Ala Ser Ala Ile Glu Trp Ala Leu Thr Glu Leu Leu
                325                 330                 335

Arg Ser Pro Glu Asp Leu Lys Arg Val Gln Gln Glu Leu Ala Glu Val
            340                 345                 350

Val Gly Leu Asp Arg Arg Val Glu Glu Ser Asp Ile Glu Lys Leu Thr
        355                 360                 365

Tyr Leu Lys Cys Thr Leu Lys Glu Thr Leu Arg Met His Pro Pro Ile
    370                 375                 380

Pro Leu Leu Leu His Glu Thr Ala Glu Asp Thr Ser Ile Asp Gly Phe
385                 390                 395                 400

Phe Ile Pro Lys Lys Ser Arg Val Met Ile Asn Ala Phe Ala Ile Gly
                405                 410                 415

Arg Asp Pro Thr Ser Trp Thr Asp Pro Asp Thr Phe Arg Pro Ser Arg
            420                 425                 430

Phe Leu Glu Pro Gly Val Pro Asp Phe Lys Gly Ser Asn Phe Glu Phe

435                 440                 445

Ile Pro Phe Gly Ser Gly Arg Arg Ser Cys Pro Gly Met Gln Leu Gly
    450                 455                 460

Leu Tyr Ala Leu Asp Leu Ala Val Ala His Ile Leu His Cys Phe Thr
465                 470                 475                 480

Trp Lys Leu Pro Asp Gly Met Lys Pro Ser Glu Leu Asp Met Asn Asp
                485                 490                 495

Val Phe Gly Leu Thr Ala Pro Lys Ala Thr Arg Leu Phe Ala Val Pro
            500                 505                 510

Thr Thr Arg Leu Ile Cys Ala Leu
        515                 520

<210>  3
<211>  1545
<212>  DNA
<213>  Camptotheca acuminata

<400>  3
atggaaatgg atgctcttcg ccaagttttg catcctatgc ccacagctct cttcttcatt      60

gcccctttgt tcttcttgtt tattctatct cgccttcgcc gaaaactacc atatccaccg     120

gggccaaacg gcttaccact tgtcggaaac atgatgatga tggaccagtt aactcaccgc     180

ggactcgcca aactcgccaa gcaatacggt ggcatctttc accttcgaat gggatttctt     240

cacatggtgg ccgtgtccag cccagaaatc gcccgccagg ttcttcaggt ccaagacaat     300

atcttctcga atcgtccggc gaccatagcc atcagctacc tcacctacga ccgcgccgac     360

atggcttttg ctcactacgg ccctttttgg aggcagatgc ggaagctatg cgtcatgaag     420

cttttcagcc gcaaacgtgc tgagtcgtgg gagtccgtac gagacgaggt tgattcgatg     480

gtccgagtgg tggtgtccca caccgggaaa tctgtgaaca taggcgagtt agtgttttca     540

ctgactcggg atattattta ccgagctgcg ttcggttcga gctcgcatga aggccaggac     600

gagttcatca agatattgca ggaattctcg aaattgtttg gtgctttcaa tatcgctgat     660

ttcattcctg gactggggtg ggttgatcct cagggactca acgccagact ggttcaggct     720

cgtgcatcgc tcgatggctt cattgactca atcattgatg atcatatcga gaagcataga     780

agaagaaacg tcgtcgactc tgatggtgat atggtggatg atttattagc tttttacagc     840

gatgaggcaa aagtcactga atccgacgat ttacagaacg ccatcagact cacaagagat     900

aatatcaagg ccatcatcat ggacgtgatg tttggaggga ctgaaacagt ggcatcggca     960

atcgagtggg ccatggcaga actaatgaaa agcccagaag accttaagag agtgcaacaa    1020

gaactcgccg aggttgtagg tctcgaccgg agactcgaag agagcgactt tgacaagcta 1080

acatacctca aatgtgcgtt aaaagagacc ctccgactgc acccgccgat tcctctcctc 1140

ctccacgaga ccgccgagga cacggaggtt gcgggctact atatccccgc gaggtcgcgt 1200

gtcatgatca acgcatgggc cattggacgc gacaagaact cgtgggacga acccgagacc 1260

ttcaagcctt ctaggttcct caaagaaggc gtgccggact ttaaaggaag caacttcgaa 1320

ttcattccat ttgggtcggg tcggaggtcg tgccccggaa tgcaactcgg actctacgcg 1380

ctcgagatgg ccgtcgctca ccttcttcac tgttttacgt ggacattgcc ggacgggatg 1440

aaaccaagtg aactggacac cgacgatgtg tttggactca ctgctccgag agcgactctt 1500

tttgtcgccg ttccgagccc acgtttactg tgtccactct attga 1545

<210> 4
<211> 514
<212> PRT
<213> Camptotheca acuminata

<400> 4

Met Glu Met Asp Ala Leu Arg Gln Val Leu His Pro Met Pro Thr Ala
1               5                   10                  15

Leu Phe Phe Ile Ala Pro Leu Phe Phe Leu Phe Ile Leu Ser Arg Leu
            20                  25                  30

Arg Arg Lys Leu Pro Tyr Pro Pro Gly Pro Asn Gly Leu Pro Leu Val
        35                  40                  45

Gly Asn Met Met Met Met Asp Gln Leu Thr His Arg Gly Leu Ala Lys
    50                  55                  60

Leu Ala Lys Gln Tyr Gly Gly Ile Phe His Leu Arg Met Gly Phe Leu
65                  70                  75                  80

His Met Val Ala Val Ser Ser Pro Glu Ile Ala Arg Gln Val Leu Gln
                85                  90                  95

Val Gln Asp Asn Ile Phe Ser Asn Arg Pro Ala Thr Ile Ala Ile Ser
            100                 105                 110

Tyr Leu Thr Tyr Asp Arg Ala Asp Met Ala Phe Ala His Tyr Gly Pro
        115                 120                 125

Phe Trp Arg Gln Met Arg Lys Leu Cys Val Met Lys Leu Phe Ser Arg
    130                 135                 140

Lys Arg Ala Glu Ser Trp Glu Ser Val Arg Asp Glu Val Asp Ser Met

145                 150                 155                 160

Val Arg Val Val Val Ser His Thr Gly Lys Ser Val Asn Ile Gly Glu
                165                 170                 175

Leu Val Phe Ser Leu Thr Arg Asp Ile Ile Tyr Arg Ala Ala Phe Gly
            180                 185                 190

Ser Ser Ser His Glu Gly Gln Asp Glu Phe Ile Lys Ile Leu Gln Glu
        195                 200                 205

Phe Ser Lys Leu Phe Gly Ala Phe Asn Ile Ala Asp Phe Ile Pro Gly
    210                 215                 220

Leu Gly Trp Val Asp Pro Gln Gly Leu Asn Ala Arg Leu Val Gln Ala
225                 230                 235                 240

Arg Ala Ser Leu Asp Gly Phe Ile Asp Ser Ile Ile Asp Asp His Ile
                245                 250                 255

Glu Lys His Arg Arg Arg Asn Val Val Asp Ser Asp Gly Asp Met Val
            260                 265                 270

Asp Asp Leu Leu Ala Phe Tyr Ser Asp Glu Ala Lys Val Thr Glu Ser
        275                 280                 285

Asp Asp Leu Gln Asn Ala Ile Arg Leu Thr Arg Asp Asn Ile Lys Ala
    290                 295                 300

Ile Ile Met Asp Val Met Phe Gly Gly Thr Glu Thr Val Ala Ser Ala
305                 310                 315                 320

Ile Glu Trp Ala Met Ala Glu Leu Met Lys Ser Pro Glu Asp Leu Lys
                325                 330                 335

Arg Val Gln Gln Glu Leu Ala Glu Val Val Gly Leu Asp Arg Arg Leu
            340                 345                 350

Glu Glu Ser Asp Phe Asp Lys Leu Thr Tyr Leu Lys Cys Ala Leu Lys
        355                 360                 365

Glu Thr Leu Arg Leu His Pro Pro Ile Pro Leu Leu Leu His Glu Thr
    370                 375                 380

Ala Glu Asp Thr Glu Val Ala Gly Tyr Tyr Ile Pro Ala Arg Ser Arg
385                 390                 395                 400

Val Met Ile Asn Ala Trp Ala Ile Gly Arg Asp Lys Asn Ser Trp Asp
                405                 410                 415

Glu Pro Glu Thr Phe Lys Pro Ser Arg Phe Leu Lys Glu Gly Val Pro
            420                 425                 430

Asp Phe Lys Gly Ser Asn Phe Glu Phe Ile Pro Phe Gly Ser Gly Arg
        435                 440                 445

Arg Ser Cys Pro Gly Met Gln Leu Gly Leu Tyr Ala Leu Glu Met Ala
    450                 455                 460

Val Ala His Leu Leu His Cys Phe Thr Trp Thr Leu Pro Asp Gly Met
465                 470                 475                 480

Lys Pro Ser Glu Leu Asp Thr Asp Asp Val Phe Gly Leu Thr Ala Pro
                485                 490                 495

Arg Ala Thr Leu Phe Val Ala Val Pro Ser Pro Arg Leu Leu Cys Pro
            500                 505                 510

Leu Tyr

<210>  5
<211>  1880
<212>  DNA
<213>  Brassica napus

<400>  5
ataaaaaaac attttccaga aaaaaaagaa aaactaatat ggagtcttct atatcacaaa      60

cactaagcca agtaatagat cccaccacgg gtattctcat cgtcgtctca cttttcatct     120

tcatcggcct catcacacga cgacgaaggc ctccgtatcc acccggtcca cgtggttggc     180

ccatcatagg caatatgtca atgatggacc aactcaccca ccgtggttta gccaacttag     240

ctaaaaagta cggtggcttg tgccatctcc gcatgggatt tctccacatg tacgccgttt     300

catcaccaga cgtggctaaa caagtccttc aagtccaaga cagcgtcttc tcgaaccgac     360

cagctactat agctataagc tatttgactt atgaccgagc cgacatggcg tttgctcact     420

acggaccgtt ttggagacag atgaggaaag tttgcgtcat gaaggtgttt agccgtaaac     480

gagccgagtc atgggcttct gttcgtgatg aagtggacaa aatgatccgg tcggtttcta     540

gtaacgttgg taagtctatc aacgttggtg agcaaatttt tgcactgacc cgaaacataa     600

cttaccgggc agcgttcggg tcagcatgtg aaaagggaca agacgagttc ataagaattt     660

tacaagagtt ctctaagctt tttggagcct tcaacgtagc ggatttcata ccgtatttcg     720

ggtggatcga tcctcaagga ataaacaagc ggctcgtgaa ggcccgtaat gacctagacg     780

gatttattga cgatatcatc gatgaacaca tgaagaagaa agagaatcaa aacagtgttg 840

atgctggaga tgttgttgat accgatatgg ttgatgatct tcttgctttt tacagtgaag 900

aggcgaaatt agtgagcgag acagcggatc ttcagaactc tatcaaactt acccgtgaca 960

atatcaaagc aatcatcatg gacgttatgt ttggaggaac ggaaacggta gcgtcagcga 1020

tagagtgggc attgactgaa ttactacgga gccccgagga tctaaaccga gtccaacaag 1080

aactcgctga agtcgtcgga cttgaccgac gtgtggaaga atcagacatc gagaagttga 1140

cttttctgaa atgcacactc aaagaaaccc taaggctaca cccaccgatc ccactcctcc 1200

tacacgaaac cgcagaggac actgagatcg acggttactt cgttcccaag aaatctcgcg 1260

ttatgatcaa cgcgtttgcg attgggcgcg acaagaactc ttgggttgac cccgaaacgt 1320

ttagaccgtc taggtttttg gaaccgggcg taccagattt caaagggagt aacttcgagt 1380

ttataccatt cgggtcgggt cgtaggtcgt gcccgggtat gcaactcggg ttatacgcgc 1440

ttgaactagc cgtggcccat atattacatt gcttcacgtg gaaattacct gatggcatga 1500

aaccaagcga gcttgatatg agcgacgtgt ttggtctgac ggctcctaaa gccacgcgtc 1560

tctatgctgt cccgagcacg cgccttattt gttctgttta agttatggtt cgaagcacgt 1620

ggcgggtgaa aggaaaggtg gttggtatgg ttcttgaaag tggtgtgaga agtcaaaaga 1680

agccctgaag atttgtggat gttatatata atatatgttt atgtatttgt gtgttcacac 1740

gtgtgttctg gatgaaacat aaagtggctc tttgtttcgt tttccaattt cttttgtggg 1800

aattcttttc cttgcatgaa atgtaaacgc tgaaaaataa gatttttttt tacaactaaa 1860

aaaaaaaaaa aaaaaaaaaa 1880

<210> 6
<211> 520
<212> PRT
<213> Brassica napus

<400> 6

Met Glu Ser Ser Ile Ser Gln Thr Leu Ser Gln Val Ile Asp Pro Thr
1               5                   10                  15

Thr Gly Ile Leu Ile Val Val Ser Leu Phe Ile Phe Ile Gly Leu Ile
            20                  25                  30

Thr Arg Arg Arg Arg Pro Pro Tyr Pro Pro Gly Pro Arg Gly Trp Pro
        35                  40                  45

Ile Ile Gly Asn Met Ser Met Met Asp Gln Leu Thr His Arg Gly Leu
    50                  55                  60

Ala Asn Leu Ala Lys Lys Tyr Gly Gly Leu Cys His Leu Arg Met Gly
65                  70                  75                  80

Phe Leu His Met Tyr Ala Val Ser Ser Pro Asp Val Ala Lys Gln Val
                85                  90                  95

Leu Gln Val Gln Asp Ser Val Phe Ser Asn Arg Pro Ala Thr Ile Ala
            100                 105                 110

Ile Ser Tyr Leu Thr Tyr Asp Arg Ala Asp Met Ala Phe Ala His Tyr
        115                 120                 125

Gly Pro Phe Trp Arg Gln Met Arg Lys Val Cys Val Met Lys Val Phe
    130                 135                 140

Ser Arg Lys Arg Ala Glu Ser Trp Ala Ser Val Arg Asp Glu Val Asp
145                 150                 155                 160

Lys Met Ile Arg Ser Val Ser Ser Asn Val Gly Lys Ser Ile Asn Val
                165                 170                 175

Gly Glu Gln Ile Phe Ala Leu Thr Arg Asn Ile Thr Tyr Arg Ala Ala
            180                 185                 190

Phe Gly Ser Ala Cys Glu Lys Gly Gln Asp Glu Phe Ile Arg Ile Leu
        195                 200                 205

Gln Glu Phe Ser Lys Leu Phe Gly Ala Phe Asn Val Ala Asp Phe Ile
    210                 215                 220

Pro Tyr Phe Gly Trp Ile Asp Pro Gln Gly Ile Asn Lys Arg Leu Val
225                 230                 235                 240

Lys Ala Arg Asn Asp Leu Asp Gly Phe Ile Asp Asp Ile Ile Asp Glu
                245                 250                 255

His Met Lys Lys Lys Glu Asn Gln Asn Ser Val Asp Ala Gly Asp Val
            260                 265                 270

Val Asp Thr Asp Met Val Asp Asp Leu Leu Ala Phe Tyr Ser Glu Glu
        275                 280                 285

Ala Lys Leu Val Ser Glu Thr Ala Asp Leu Gln Asn Ser Ile Lys Leu
    290                 295                 300

Thr Arg Asp Asn Ile Lys Ala Ile Ile Met Asp Val Met Phe Gly Gly
305                 310                 315                 320

Thr Glu Thr Val Ala Ser Ala Ile Glu Trp Ala Leu Thr Glu Leu Leu
                325                 330                 335

Arg Ser Pro Glu Asp Leu Asn Arg Val Gln Gln Glu Leu Ala Glu Val
            340                 345                 350

Val Gly Leu Asp Arg Arg Val Glu Glu Ser Asp Ile Glu Lys Leu Thr
        355                 360                 365

Phe Leu Lys Cys Thr Leu Lys Glu Thr Leu Arg Leu His Pro Pro Ile
    370                 375                 380

Pro Leu Leu Leu His Glu Thr Ala Glu Asp Thr Glu Ile Asp Gly Tyr
385                 390                 395                 400

Phe Val Pro Lys Lys Ser Arg Val Met Ile Asn Ala Phe Ala Ile Gly
                405                 410                 415

Arg Asp Lys Asn Ser Trp Val Asp Pro Glu Thr Phe Arg Pro Ser Arg
            420                 425                 430

Phe Leu Glu Pro Gly Val Pro Asp Phe Lys Gly Ser Asn Phe Glu Phe
        435                 440                 445

Ile Pro Phe Gly Ser Gly Arg Arg Ser Cys Pro Gly Met Gln Leu Gly
    450                 455                 460

Leu Tyr Ala Leu Glu Leu Ala Val Ala His Ile Leu His Cys Phe Thr
465                 470                 475                 480

Trp Lys Leu Pro Asp Gly Met Lys Pro Ser Glu Leu Asp Met Ser Asp
                485                 490                 495

Val Phe Gly Leu Thr Ala Pro Lys Ala Thr Arg Leu Tyr Ala Val Pro
            500                 505                 510

Ser Thr Arg Leu Ile Cys Ser Val
        515                 520

<210>  7
<211>  1857
<212>  DNA
<213>  Glycine max

<400>  7
ggaattcggc acgagggcga aatcatccaa acacattcca tctattaaga ttttagagag      60

tgaaaatgga ttggcaaagc atgatgggca acctggaccc attccaaaga acaatcttaa     120

tcctcgtccc actaacacta ctcctgctgc tgttactatc tcgtacccgt ccaagacccc 180

cctatccacc aggccctaag ggcttcccaa tcataggaaa catgtttatg atggaccagc 240

taacccaccg cggcctcgcc aacctggcca aacactacgg cggaatcttc cacctccgca 300

tgggcttcct ccacatggtc gccatctccg accccgacgc cgcgcgacag gttctccaag 360

tccaagacaa catcttctcc aaccgcccag ccaccatcgc catcagctac cttacctacg 420

accgcgccga catggctttt gcccactacg gccccttctg gcgccagatg cggaaactct 480

gcgtcatgaa gctcttcagc cgcaagcgcg ccgagtcctg gcagtcagtc cgcgatgagg 540

tcgacgccgc cgttcgcgcc gtcgctagca gcgtcggaaa gcccgtcaac attggagaat 600

tagtgtttaa cctcaccaag aacatcatct accgcgccgc gttcgggtcg agttcccaag 660

aaggccagga cgagttcatt aaaatactgc aggagttctc caagctcttt ggcgcgttta 720

atattgcgga ttttataccc tatctcgggt gcgtggatcc acaaggtttg aactcgagac 780

tcgctagggc acgtggcgcg ctcgatagct tcattgataa gatcatcgac gagcacgtgc 840

ataagatgaa gaatgataag agcagtgaaa ttgttgatgg agaaacggac atggtggatg 900

agttgctggc gttctacagc gaggaggcga agttgaacaa tgaatcggac gatttgcaga 960

actctatcag actcactaag gataacatca aggctatcat tatggacgtg atgttcggag 1020

gcacggaaac ggtagcgtca gcgatcgagt gggccatggc ggagctcatg agaagcccag 1080

aagatcaaaa gcgggtccaa caagagctgg cggatgtagt gggcctggac cgtcgggccg 1140

aagagtccga tttcgagaaa ctcacttatc tcaaatgtgc cctcaaagag accctccgcc 1200

tccaccctcc gataccgctc ctcctccacg agacggcgga ggacgccacc gtcggcggct 1260

acttcgtccc caggaaggcg cgtgttatga tcaacgcgtg ggccattggg agggacaaga 1320

attcctggga ggaacccgaa acttttaagc ccgcccggtt ccttaaaccg ggcgtgcccg 1380

atttcaaggg gagcaacttc gagttcattc cattcgggtc gggtcgaaga tcctgccccg 1440

gaatggtgtt ggggctctac gcgcttgaat tggcggtggc tcatcttctt cactgcttca 1500

cgtgggaatt gccagatgga atgaaaccaa gtgagatgga catgggtgac gtgttcggac 1560

tcaccgctcc aaggtccacg cgactcattg ctgtaccaac caagcgcgtg gtgtgccctc 1620

tcttttaaaa ataaaaaaaa aacacttgga tctttctttt tttcctcttt gtattttctt 1680

tttatccctt aattatacat aatcataata aatgatataa agggaaaaaa gggaggtgcg 1740

caataagaat gtgggagtat ttggaagaaa aaaatttgta aaactcttct tgatcattga 1800

tgtgagcttt tgtgcagaaa caattaatta tgtatgtatt ttgtttcttt ttgcctc 1857

<210> 8
<211> 520
<212> PRT
<213> Glycine max

<400>  8

Met Asp Trp Gln Ser Met Met Gly Asn Leu Asp Pro Phe Gln Arg Thr
1               5                   10                  15

Ile Leu Ile Leu Val Pro Leu Thr Leu Leu Leu Leu Leu Leu Leu Ser
            20                  25                  30

Arg Thr Arg Pro Arg Pro Pro Tyr Pro Pro Gly Pro Lys Gly Phe Pro
        35                  40                  45

Ile Ile Gly Asn Met Phe Met Met Asp Gln Leu Thr His Arg Gly Leu
    50                  55                  60

Ala Asn Leu Ala Lys His Tyr Gly Gly Ile Phe His Leu Arg Met Gly
65                  70                  75                  80

Phe Leu His Met Val Ala Ile Ser Asp Pro Asp Ala Ala Arg Gln Val
                85                  90                  95

Leu Gln Val Gln Asp Asn Ile Phe Ser Asn Arg Pro Ala Thr Ile Ala
            100                 105                 110

Ile Ser Tyr Leu Thr Tyr Asp Arg Ala Asp Met Ala Phe Ala His Tyr
        115                 120                 125

Gly Pro Phe Trp Arg Gln Met Arg Lys Leu Cys Val Met Lys Leu Phe
    130                 135                 140

Ser Arg Lys Arg Ala Glu Ser Trp Gln Ser Val Arg Asp Glu Val Asp
145                 150                 155                 160

Ala Ala Val Arg Ala Val Ala Ser Ser Val Gly Lys Pro Val Asn Ile
                165                 170                 175

Gly Glu Leu Val Phe Asn Leu Thr Lys Asn Ile Ile Tyr Arg Ala Ala
            180                 185                 190

Phe Gly Ser Ser Ser Gln Glu Gly Gln Asp Glu Phe Ile Lys Ile Leu
        195                 200                 205

Gln Glu Phe Ser Lys Leu Phe Gly Ala Phe Asn Ile Ala Asp Phe Ile
    210                 215                 220

Pro Tyr Leu Gly Cys Val Asp Pro Gln Gly Leu Asn Ser Arg Leu Ala
225                 230                 235                 240

Arg Ala Arg Gly Ala Leu Asp Ser Phe Ile Asp Lys Ile Ile Asp Glu
                245                 250                 255

His Val His Lys Met Lys Asn Asp Lys Ser Ser Glu Ile Val Asp Gly
            260                 265                 270

Glu Thr Asp Met Val Asp Glu Leu Leu Ala Phe Tyr Ser Glu Glu Ala
        275                 280                 285

Lys Leu Asn Asn Glu Ser Asp Asp Leu Gln Asn Ser Ile Arg Leu Thr
    290                 295                 300

Lys Asp Asn Ile Lys Ala Ile Ile Met Asp Val Met Phe Gly Gly Thr
305                 310                 315                 320

Glu Thr Val Ala Ser Ala Ile Glu Trp Ala Met Ala Glu Leu Met Arg
                325                 330                 335

Ser Pro Glu Asp Gln Lys Arg Val Gln Gln Glu Leu Ala Asp Val Val
            340                 345                 350

Gly Leu Asp Arg Arg Ala Glu Glu Ser Asp Phe Glu Lys Leu Thr Tyr
        355                 360                 365

Leu Lys Cys Ala Leu Lys Glu Thr Leu Arg Leu His Pro Pro Ile Pro
    370                 375                 380

Leu Leu Leu His Glu Thr Ala Glu Asp Ala Thr Val Gly Gly Tyr Phe
385                 390                 395                 400

Val Pro Arg Lys Ala Arg Val Met Ile Asn Ala Trp Ala Ile Gly Arg
                405                 410                 415

Asp Lys Asn Ser Trp Glu Glu Pro Glu Thr Phe Lys Pro Ala Arg Phe
            420                 425                 430

Leu Lys Pro Gly Val Pro Asp Phe Lys Gly Ser Asn Phe Glu Phe Ile
        435                 440                 445

Pro Phe Gly Ser Gly Arg Arg Ser Cys Pro Gly Met Val Leu Gly Leu
    450                 455                 460

Tyr Ala Leu Glu Leu Ala Val Ala His Leu Leu His Cys Phe Thr Trp
465                 470                 475                 480

Glu Leu Pro Asp Gly Met Lys Pro Ser Glu Met Asp Met Gly Asp Val
                485                 490                 495

Phe Gly Leu Thr Ala Pro Arg Ser Thr Arg Leu Ile Ala Val Pro Thr
            500                 505                 510

Lys Arg Val Val Cys Pro Leu Phe
        515                 520

<210>  9
<211>  1960
<212>  DNA
<213>  Artificial Sequence

<220>
<223>  Lycopersicon esculentum  x  Lycopersicon peruvianum

<400>  9
aaagccattg taaacaaaat gaaagaaatg gtactaaaca atatcaattc aattcttgaa      60

gctttacaag ctaaccctat actaatcttc ttcttcatca tccctctctt cttcttatac     120

cttttttcga cgtctcgccg taaacgttat cctccaggac cattaggctg gcctctcatt     180

ggtaacatga tgataatgga ccagttaact caccgtggtc ttgccaaact tgcacaaaaa     240

tatggtggcg tttttcacct caaaatgggt tatgtccaca aaattgttat atctggtcca     300

gaagaagctc gtcaagtact acaggtacaa gacaacatat attcgaatcg tccaaagacc     360

gtagcgataa gttacctaac gtacgatcgt gcggacatgg cttttgctga ctacggaccg     420

ttttggcgtc agatgagaaa attatgtgtt atgaaattat tcagtcgtaa acgagctgag     480

tcatgggact cggttcgcga cgaggtcgac tcaatggtaa agatagttac aaccaacgcg     540

ggcacatcaa ttaacttagg ggaacttgtt ttctgtctca ctcgtaacat tatctatcga     600

gctgcttttg ggacaagttc agatgaagga caagatgatt ttattaaaat tttgcaagaa     660

ttttcgaagc tatttggtgc ttttaacatg gctgatttta ttccatggct agggtggatt     720

ggtaagcaag gcctaaatgt tagacttgct aaggcaaggg catcacttga tggatttatt     780

gatacgataa ttgatgatca tattgaaaga aagaaggcta ttcatgttat taatgatgat     840

ggttatagag aaagtgatat ggtggatgag cttttagctt tttacagtga agaaacaaaa     900

gtaaatgagt ctgaagattt gcagaatgct ataaggctta ctagggataa tatcaaagct     960

ataatcatgg atgtaatgtt tggtgggacc gagacggtgg catctgcgat agagtgggcc    1020

atggctgagc ttatgaaaag tccagaggat cttaaaaagg tacaacaaga attagctaac    1080

gttgttggac taaataggaa agttgatgaa tccgattttg aaaacttgac atacttaaag    1140

tgttgtttaa aagaaactct tcgactccac cctccaatac ctctcctcct ccacgaaacc    1200

gcggaggaat cgacagtctc cggctactat attccagcaa actcacatgt tatcataaat    1260

tcgtttgcaa ttggacgtga caaaaactca tgggaagatc ctgatagttt caagccaagt    1320

aggttcctta aagaaggtgt ggcagatttt aaaggtggta attttgaatt tttaccattt    1380

gggtcgggtc gaaggtcttg ccccggtatg caacttgggc tttatgcatt agaaatggct 1440

gtggcccatt tacttcattg ttttacttgg gaattacctg atggaatgaa accaagtgaa 1500

ctaaatatgg atgatatgtt tgggcttact gctccattgg ccaatcgatt agtggctgtg 1560

cctactccac gtttattatg tgaaatttat tgaagaccaa aagggggtat tttgtacgtg 1620

tatacgaata gtttatacac aaaatattgc tttgtttgga catttttatc tttattttgt 1680

tttgtctatg catatataca tgatggattg gagagggaca aaagaaaaag gacgaaaata 1740

attgcttgaa aggggaaaga gagaggaatt gattgtatgg catgcatttt gttatttaat 1800

attcatttgg cttgcattgc tagttgtaaa ttaaatgtcc cctcctatgg tttgtttctt 1860

ctttgaaaac ataattttta aatcaaataa agattttatc tttgttccct taaaaaaaaa 1920

aaaaaaaaaa aaaaaaaaaa aaaaaaaaaa aaaaaaaaaa 1960

<210> 10
<211> 521
<212> PRT
<213> Artificial Sequence

<220>
<223> Lycopersicon esculentum x Lycopersicon peruvianum

<400> 10

Met Val Leu Asn Asn Ile Asn Ser Ile Leu Glu Ala Leu Gln Ala Asn
1               5                   10                  15

Pro Ile Leu Ile Phe Phe Phe Ile Ile Pro Leu Phe Phe Leu Tyr Leu
            20                  25                  30

Phe Ser Thr Ser Arg Arg Lys Arg Tyr Pro Pro Gly Pro Leu Gly Trp
        35                  40                  45

Pro Leu Ile Gly Asn Met Met Ile Met Asp Gln Leu Thr His Arg Gly
    50                  55                  60

Leu Ala Lys Leu Ala Gln Lys Tyr Gly Gly Val Phe His Leu Lys Met
65                  70                  75                  80

Gly Tyr Val His Lys Ile Val Ile Ser Gly Pro Glu Glu Ala Arg Gln
                85                  90                  95

Val Leu Gln Val Gln Asp Asn Ile Tyr Ser Asn Arg Pro Lys Thr Val
            100                 105                 110

Ala Ile Ser Tyr Leu Thr Tyr Asp Arg Ala Asp Met Ala Phe Ala Asp
        115                 120                 125

Tyr Gly Pro Phe Trp Arg Gln Met Arg Lys Leu Cys Val Met Lys Leu
    130                 135                 140

Phe Ser Arg Lys Arg Ala Glu Ser Trp Asp Ser Val Arg Asp Glu Val
145                 150                 155                 160

Asp Ser Met Val Lys Ile Val Thr Thr Asn Ala Gly Thr Ser Ile Asn
                165                 170                 175

Leu Gly Glu Leu Val Phe Cys Leu Thr Arg Asn Ile Ile Tyr Arg Ala
            180                 185                 190

Ala Phe Gly Thr Ser Ser Asp Glu Gly Gln Asp Asp Phe Ile Lys Ile
        195                 200                 205

Leu Gln Glu Phe Ser Lys Leu Phe Gly Ala Phe Asn Met Ala Asp Phe
    210                 215                 220

Ile Pro Trp Leu Gly Trp Ile Gly Lys Gln Gly Leu Asn Val Arg Leu
225                 230                 235                 240

Ala Lys Ala Arg Ala Ser Leu Asp Gly Phe Ile Asp Thr Ile Ile Asp
                245                 250                 255

Asp His Ile Glu Arg Lys Lys Ala Ile His Val Ile Asn Asp Asp Gly
            260                 265                 270

Tyr Arg Glu Ser Asp Met Val Asp Glu Leu Leu Ala Phe Tyr Ser Glu
        275                 280                 285

Glu Thr Lys Val Asn Glu Ser Glu Asp Leu Gln Asn Ala Ile Arg Leu
    290                 295                 300

Thr Arg Asp Asn Ile Lys Ala Ile Ile Met Asp Val Met Phe Gly Gly
305                 310                 315                 320

Thr Glu Thr Val Ala Ser Ala Ile Glu Trp Ala Met Ala Glu Leu Met
                325                 330                 335

Lys Ser Pro Glu Asp Leu Lys Lys Val Gln Gln Glu Leu Ala Asn Val
            340                 345                 350

Val Gly Leu Asn Arg Lys Val Asp Glu Ser Asp Phe Glu Asn Leu Thr
        355                 360                 365

Tyr Leu Lys Cys Cys Leu Lys Glu Thr Leu Arg Leu His Pro Pro Ile
    370                 375                 380

Pro Leu Leu Leu His Glu Thr Ala Glu Glu Ser Thr Val Ser Gly Tyr
385                 390                 395                 400

Tyr Ile Pro Ala Asn Ser His Val Ile Ile Asn Ser Phe Ala Ile Gly
                405                 410                 415

Arg Asp Lys Asn Ser Trp Glu Asp Pro Asp Ser Phe Lys Pro Ser Arg
            420                 425                 430

Phe Leu Lys Glu Gly Val Ala Asp Phe Lys Gly Gly Asn Phe Glu Phe
        435                 440                 445

Leu Pro Phe Gly Ser Gly Arg Arg Ser Cys Pro Gly Met Gln Leu Gly
    450                 455                 460

Leu Tyr Ala Leu Glu Met Ala Val Ala His Leu Leu His Cys Phe Thr
465                 470                 475                 480

Trp Glu Leu Pro Asp Gly Met Lys Pro Ser Glu Leu Asn Met Asp Asp
                485                 490                 495

Met Phe Gly Leu Thr Ala Pro Leu Ala Asn Arg Leu Val Ala Val Pro
            500                 505                 510

Thr Pro Arg Leu Leu Cys Glu Ile Tyr
        515                 520

<210>  11
<211>  1885
<212>  DNA
<213>  Medicago sativa

<400>  11
ggcacgaggc attcaaaccc aaaaaactat aggaatattc acaatcatat agctgcataa      60

attaacacaa aaccatatgg attctcttct aaaatatcca atcatggaga actttaagga     120

agaaccattt cttatggcaa tcatgttcat cttaccacta atactcttgt tgggtttagt     180

gtcacgaatc ctcaaaaggc caagatatcc accaggacca aaagggttac ctgttatagg     240

taacatgcta atgatggacc aattaaccca ccgtggtcta gccaacttag ccaaaaaata     300

tggaggcatc tttcacctac gcatgggatt cctccacatg gtagctattt ccgacgcaga     360

cgccgcacga caagttctcc aagttcaaga caacatcttt tccaaccggc cagcaactgt     420

agccattaaa tacctaactt acgaccgtgc tgacatggcg ttcgctcact acggtccctt     480

ttggcgccag atgcgaaaac tttgcgtgat gaagcttttc agccgcaagc acgcagagtc     540

ttggcaatct gttagagacg aggttgacca tgctatccga actgtttcgg acaacatagg     600

caaccctgtg aacattggag aactggtgtt caatttaact aaaaacatta tatatcgagc 660

ggctttcggg tcgagctcaa gagaaggaca agatgagttt attggaatat tgcaagagtt 720

ttccaaattg tttggagctt ttaatatttc cgactttgta ccttgttttg gagctattga 780

ccctcaaggg cttaatgcta ggcttgtgaa ggctcgtaaa gatttggata gtttcataga 840

caaaatcata gatgaacatg tgcagaagaa gaaaagtgtt gttgatgaag aaacggatat 900

ggtggatgag ttgcttgctt tctatagtga ggaggctaaa ttgaataatg aatcagatga 960

tttgcataat tccatcaaac ttaccaagga taacatcaaa gccatcataa tggacgtgat 1020

gtttggagga acggaaacgg tagcatcagc aatcgaatgg gctatggcag agttaatgaa 1080

aagcccagaa gatcttaaaa aagtccaaca agaactagca gaagttgtcg gcctgagccg 1140

acaggtcgaa gagcccgatt tcgagaaact aacttatctg aaatgcgctc ttaaggaaac 1200

cctacgtctt cacccaccaa ttcctttgct tcttcacgaa actgcggaag aagcaacggt 1260

taatggttat ttcattccaa agcaagcgcg tgtgatgata aacgcatggg ctattggaag 1320

agacgcaaat tgttgggaag aacccgagag ttttaagcca tcacggtttt tgaaaccagg 1380

tgtgcccgat tttaaaggga gtaattttga gtttattccg tttgggtcag gacgtagatc 1440

ctgtccaggt atgcagttgg gtttgtacgc gcttgatttg gcggtagctc atttacttca 1500

ttgctttact tgggagttgc cggatggaat gaaaccaagt gagatggata tgagtgatgt 1560

atttggactc actgctccga gagcaagtcg actcattgct attcctacta agcgtgtctt 1620

gtgtcctctg gattagaaag aaaaagagga agaaaaaaaa ttgaccttga attttctatt 1680

ttgagttttt cttttttatt gttattctat taaacaataa gcataagtgt tatgtgatga 1740

taaaataaga cacgtaacaa gaacgtgaga gtgtaaaaga attggaattt tcttcacttt 1800

tcttgatgta agcttctatt ttcaaatatt ataatgtatt atcctataag aaacaattcc 1860

ttgaataaaa aaaaaaaaaa aaaaa 1885

<210> 12
<211> 519
<212> PRT
<213> Medicago sativa

<400> 12

Met Asp Ser Leu Leu Lys Tyr Pro Ile Met Glu Asn Phe Lys Glu Glu
1               5                   10                  15

Pro Phe Leu Met Ala Ile Met Phe Ile Leu Pro Leu Ile Leu Leu Leu
            20                  25                  30

Gly Leu Val Ser Arg Ile Leu Lys Arg Pro Arg Tyr Pro Pro Gly Pro
        35                  40                  45

Lys Gly Leu Pro Val Ile Gly Asn Met Leu Met Met Asp Gln Leu Thr
    50                  55                  60

His Arg Gly Leu Ala Asn Leu Ala Lys Lys Tyr Gly Gly Ile Phe His
65                  70                  75                  80

Leu Arg Met Gly Phe Leu His Met Val Ala Ile Ser Asp Ala Asp Ala
                85                  90                  95

Ala Arg Gln Val Leu Gln Val Gln Asp Asn Ile Phe Ser Asn Arg Pro
            100                 105                 110

Ala Thr Val Ala Ile Lys Tyr Leu Thr Tyr Asp Arg Ala Asp Met Ala
        115                 120                 125

Phe Ala His Tyr Gly Pro Phe Trp Arg Gln Met Arg Lys Leu Cys Val
    130                 135                 140

Met Lys Leu Phe Ser Arg Lys His Ala Glu Ser Trp Gln Ser Val Arg
145                 150                 155                 160

Asp Glu Val Asp His Ala Ile Arg Thr Val Ser Asp Asn Ile Gly Asn
                165                 170                 175

Pro Val Asn Ile Gly Glu Leu Val Phe Asn Leu Thr Lys Asn Ile Ile
            180                 185                 190

Tyr Arg Ala Ala Phe Gly Ser Ser Ser Arg Glu Gly Gln Asp Glu Phe
        195                 200                 205

Ile Gly Ile Leu Gln Glu Phe Ser Lys Leu Phe Gly Ala Phe Asn Ile
    210                 215                 220

Ser Asp Phe Val Pro Cys Phe Gly Ala Ile Asp Pro Gln Gly Leu Asn
225                 230                 235                 240

Ala Arg Leu Val Lys Ala Arg Lys Asp Leu Asp Ser Phe Ile Asp Lys
                245                 250                 255

Ile Ile Asp Glu His Val Gln Lys Lys Lys Ser Val Val Asp Glu Glu
            260                 265                 270

Thr Asp Met Val Asp Glu Leu Leu Ala Phe Tyr Ser Glu Glu Ala Lys
        275                 280                 285

Leu Asn Asn Glu Ser Asp Asp Leu His Asn Ser Ile Lys Leu Thr Lys

290                 295                 300

Asp Asn Ile Lys Ala Ile Ile Met Asp Val Met Phe Gly Gly Thr Glu
305                 310                 315                 320

Thr Val Ala Ser Ala Ile Glu Trp Ala Met Ala Glu Leu Met Lys Ser
                325                 330                 335

Pro Glu Asp Leu Lys Lys Val Gln Gln Glu Leu Ala Glu Val Val Gly
            340                 345                 350

Leu Ser Arg Gln Val Glu Glu Pro Asp Phe Glu Lys Leu Thr Tyr Leu
        355                 360                 365

Lys Cys Ala Leu Lys Glu Thr Leu Arg Leu His Pro Pro Ile Pro Leu
    370                 375                 380

Leu Leu His Glu Thr Ala Glu Glu Ala Thr Val Asn Gly Tyr Phe Ile
385                 390                 395                 400

Pro Lys Gln Ala Arg Val Met Ile Asn Ala Trp Ala Ile Gly Arg Asp
                405                 410                 415

Ala Asn Cys Trp Glu Glu Pro Glu Ser Phe Lys Pro Ser Arg Phe Leu
            420                 425                 430

Lys Pro Gly Val Pro Asp Phe Lys Gly Ser Asn Phe Glu Phe Ile Pro
        435                 440                 445

Phe Gly Ser Gly Arg Arg Ser Cys Pro Gly Met Gln Leu Gly Leu Tyr
    450                 455                 460

Ala Leu Asp Leu Ala Val Ala His Leu Leu His Cys Phe Thr Trp Glu
465                 470                 475                 480

Leu Pro Asp Gly Met Lys Pro Ser Glu Met Asp Met Ser Asp Val Phe
                485                 490                 495

Gly Leu Thr Ala Pro Arg Ala Ser Arg Leu Ile Ala Ile Pro Thr Lys
            500                 505                 510

Arg Val Leu Cys Pro Leu Asp
        515

<210>  13
<211>  1533
<212>  DNA
<213>  Medicago truncatula

<400>  13
atggatacac taggacaatt ccaaacaact ctattattag ttctcccact cactttgttt      60

ctactactaa atttagcatc aaaatttcgt aaaagagcac catatccacc tggaccaaaa     120

gggttaccct taataggcaa catgaacatg ttggacaaac tcacacatag aggcttagca     180

aatttagcaa aacaatatgg cggtgtttat cacctccgca tggggtttat ccacatggtg     240

gcaatttcaa acgccgaagc agcgcgtgaa gtgctacaat tacacgatag catcttttca     300

aatcggccag cgacggtagc cataagttac cttacttata accgtgctga catggcgttt     360

gctcactacg gtccatttgg gcgccagatg cgaaaacttt gtgtcatgaa actctttagc     420

cgcaaacgcg ctgagtcatg gcagtctgtc aaagacgaag ttgaagttgt tatcaccaat     480

gttaacaaca atttgggaaa gtccgttaat gttggtgagt tggtttttaa tttgacaaaa     540

aatattatct atcgcgcggc ttttgggtcg tgctcgaaag aaggacaaga tgagttcatt     600

tcgatacttc aagagttttc gaaattgttt ggtgctttta atattgcgga ttttgttcct     660

tgtttgaagt gggttgatcc tcaaggtttt aatgataggc ttgttaaggc tcgtggtgcg     720

ttggatggtt ttattgataa gatttttgat gaacatgtag agaaaaaaag gaatatgagt     780

ggtgatgaag atagtgatat ggttgatgaa ttgttggctt tttacagtga tgaagctaaa     840

gtagaaaatg aatcggatga tcttcataac tccatcaaac tcaccagaga taacatcaaa     900

gccattatca tggatgtgat gttcggagga acagaaacag tagcatcagc aatggaatgg     960

gcaatgtcgg agctaatgag aaatccagaa gaactaaagc gtgtgcaaca agaactcgcc    1020

gttgttgttg gcctagaccg gcgcgtggag gaatcagaca tcgagaaact cacttatcta    1080

aagtgtgccg tcaaggagac gttgcgtctc caccctccca tcccccttct cctccacgaa    1140

accgcggagg atgcaacagt tggtggttat tttgttccta agggctcgcg cgtgatgata    1200

aatgtgtggg ctataggaag agacaaagac tcgtgggaag atccagaaga gtttaggcct    1260

tcgcggtttt tggattcaag tgcaccagat tttaaaggaa gtcattttga gtttattcca    1320

ttcgggtctg gtcgaaggtc ttgtcctgga atgcaattgg gcctttatgc gttggattta    1380

gctttggcac atttacttca ttgttttact tgggagttgc cgaatgggat gagggctagt    1440

gagatggata cgagtgatgt ttttggactc actgctccgc gagcaagtcg actcattgca    1500

gttccagcta agcgtgttgt gtgccctctc taa                                 1533


<210>  14
<211>  510
<212>  PRT
<213>  Medicago truncatula

<400>  14

Met Asp Thr Leu Gly Gln Phe Gln Thr Thr Leu Leu Leu Val Leu Pro

1               5                   10                  15

Leu Thr Leu Phe Leu Leu Leu Asn Leu Ala Ser Lys Phe Arg Lys Arg
            20                  25                  30

Ala Pro Tyr Pro Pro Gly Pro Lys Gly Leu Pro Leu Ile Gly Asn Met
        35                  40                  45

Asn Met Leu Asp Lys Leu Thr His Arg Gly Leu Ala Asn Leu Ala Lys
    50                  55                  60

Gln Tyr Gly Gly Val Tyr His Leu Arg Met Gly Phe Ile His Met Val
65                  70                  75                  80

Ala Ile Ser Asn Ala Glu Ala Ala Arg Glu Val Leu Gln Leu His Asp
                85                  90                  95

Ser Ile Phe Ser Asn Arg Pro Ala Thr Val Ala Ile Ser Tyr Leu Thr
            100                 105                 110

Tyr Asn Arg Ala Asp Met Ala Phe Ala His Tyr Gly Pro Phe Trp Arg
        115                 120                 125

Gln Met Arg Lys Leu Cys Val Met Lys Leu Phe Ser Arg Lys Arg Ala
    130                 135                 140

Glu Ser Trp Gln Ser Val Lys Asp Glu Val Glu Val Val Ile Thr Asn
145                 150                 155                 160

Val Asn Asn Asn Leu Gly Lys Ser Val Asn Val Gly Glu Leu Val Phe
                165                 170                 175

Asn Leu Thr Lys Asn Ile Ile Tyr Arg Ala Ala Phe Gly Ser Cys Ser
            180                 185                 190

Lys Glu Gly Gln Asp Glu Phe Ile Ser Ile Leu Gln Glu Phe Ser Lys
        195                 200                 205

Leu Phe Gly Ala Phe Asn Ile Ala Asp Phe Val Pro Cys Leu Lys Trp
    210                 215                 220

Val Asp Pro Gln Gly Phe Asn Asp Arg Leu Val Lys Ala Arg Gly Ala
225                 230                 235                 240

Leu Asp Gly Phe Ile Asp Lys Ile Phe Asp Glu His Val Glu Lys Lys
                245                 250                 255

Arg Asn Met Ser Gly Asp Glu Asp Ser Asp Met Val Asp Glu Leu Leu
            260                 265                 270

Ala Phe Tyr Ser Asp Glu Ala Lys Val Glu Asn Glu Ser Asp Asp Leu
        275                 280                 285

His Asn Ser Ile Lys Leu Thr Arg Asp Asn Ile Lys Ala Ile Ile Met
    290                 295                 300

Asp Val Met Phe Gly Gly Thr Glu Thr Val Ala Ser Ala Met Glu Trp
305                 310                 315                 320

Ala Met Ser Glu Leu Met Arg Asn Pro Glu Glu Leu Lys Arg Val Gln
                325                 330                 335

Gln Glu Leu Ala Val Val Val Gly Leu Asp Arg Arg Val Glu Glu Ser
            340                 345                 350

Asp Ile Glu Lys Leu Thr Tyr Leu Lys Cys Ala Val Lys Glu Thr Leu
        355                 360                 365

Arg Leu His Pro Pro Ile Pro Leu Leu Leu His Glu Thr Ala Glu Asp
    370                 375                 380

Ala Thr Val Gly Gly Tyr Phe Val Pro Lys Gly Ser Arg Val Met Ile
385                 390                 395                 400

Asn Val Trp Ala Ile Gly Arg Asp Lys Asp Ser Trp Glu Asp Pro Glu
                405                 410                 415

Glu Phe Arg Pro Ser Arg Phe Leu Asp Ser Ser Ala Pro Asp Phe Lys
            420                 425                 430

Gly Ser His Phe Glu Phe Ile Pro Phe Gly Ser Gly Arg Arg Ser Cys
        435                 440                 445

Pro Gly Met Gln Leu Gly Leu Tyr Ala Leu Asp Leu Ala Leu Ala His
    450                 455                 460

Leu Leu His Cys Phe Thr Trp Glu Leu Pro Asn Gly Met Arg Ala Ser
465                 470                 475                 480

Glu Met Asp Thr Ser Asp Val Phe Gly Leu Thr Ala Pro Arg Ala Ser
                485                 490                 495

Arg Leu Ile Ala Val Pro Ala Lys Arg Val Val Cys Pro Leu
            500                 505                 510

<210> 15
<211> 1755
<212> DNA
<213> Nicotiana tabacum

<400> 15
caacacgctt actatctcct aaatctccac tcaaaaacaa agaagagaaa gatttaaaac 60

taataattat gaaagagatg gtgcaaaaca atatgagcac ttctcttctt gaaactttac 120

aagctacgcc catgatattc tacttcatcg tccctctctt ctgcttattc cttctctcca 180

aatctcgccg taaacgtttg cctccaggtc caactggctg gcctctcatt ggtaacatga 240

tgatgatgga ccagttaact caccgtggcc ttgccaaact agcccaaaaa tatggtggtg 300

tttttcacct taaaatgggt tatgttcaca aaattgtagt ctctggtcca gacgaagctc 360

gccaagtatt acaggaacac gacatcatat tttcgaaccg tccagcgacc gtagccataa 420

gttacctaac atatgacagg gcagacatgg cttttgctga ctatggactc ttctggcggc 480

agatgagaaa actatgtgta atgaaactct tcagccgcaa acgagctgag tcatgggact 540

cagttcgaga cgaagcggat tccatggtta gaattgtaac aaccaacaca ggcacagctg 600

ttaacttagg tgaacttgtt ttcagtctca ctcgtaatat tatctacaga gctgcttttg 660

gaacttgttc tgaagatgga caaggcgagt tcattaaaat tatgcaagag ttttcgaagc 720

tatttggtgc tttcaatata gctgatttta ttccatggct agggtgggtt ggtaagcaga 780

gtctaaatat tagacttgct aaggctagag cgtcgcttga tgggttcatt gattcgatta 840

ttgatgacca tattattaga aagaaagctt atgttaatgg caaaaatgat ggaggtgatc 900

gagaaactga tatggtggat gagcttttag ctttttacag tgaggaagca aaagtaactg 960

agtccgaaga tttgcagaat gctatcagac ttactaagga taatatcaaa gctatcatca 1020

tggatgtaat gtttggaggg acagaaacag tggcttctgc aatagaatgg gccatggcag 1080

agcttatgag gagtcctgaa gatcttaaaa aggtacaaca agagctggct aacgttgttg 1140

gactcaacag aaaagttgaa gaatctgact ttgaaaaatt aacatactta agatgttgtc 1200

taaaagaaac tctacgactt caccctccaa tccctctcct cctccatgag accgccgagg 1260

aatccaccgt ctccggctac catattccgg caaagtcaca tgttattata aattcatttg 1320

ccattgggcg tgacaaaaat tcatgggaag atcctgaaac ttataaacca tctaggtttc 1380

tcaaagaagg tgtaccagat tttaaaggag gtaattttga gtttatacca tttgggtcgg 1440

gtcggcggtc ttgccccggt atgcaacttg ggctttatgc attggaaatg gctgtggccc 1500

atcttcttca ttgttttact tgggaattgc cagatggtat gaaaccaagt gagcttaaaa 1560

tggatgatat ttttggactc actgctccaa gagctaatcg actcgtggct gtgcctactc 1620

cacgtttgtt gtgtcccctt tattaattga agaaaaaagg tggggctttt acttgcatca 1680

aagagtggtg cttgtgattt ttccaccttt tggttaaata tacgaattat tatgatatac 1740

gaattcttgg gcaca 1755

<210> 16
<211> 525
<212> PRT
<213> Nicotiana tabacum

<400> 16

Met Lys Glu Met Val Gln Asn Asn Met Ser Thr Ser Leu Leu Glu Thr
1               5                   10                  15

Leu Gln Ala Thr Pro Met Ile Phe Tyr Phe Ile Val Pro Leu Phe Cys
            20                  25                  30

Leu Phe Leu Leu Ser Lys Ser Arg Arg Lys Arg Leu Pro Pro Gly Pro
        35                  40                  45

Thr Gly Trp Pro Leu Ile Gly Asn Met Met Met Met Asp Gln Leu Thr
    50                  55                  60

His Arg Gly Leu Ala Lys Leu Ala Gln Lys Tyr Gly Gly Val Phe His
65                  70                  75                  80

Leu Lys Met Gly Tyr Val His Lys Ile Val Val Ser Gly Pro Asp Glu
                85                  90                  95

Ala Arg Gln Val Leu Gln Glu His Asp Ile Ile Phe Ser Asn Arg Pro
            100                 105                 110

Ala Thr Val Ala Ile Ser Tyr Leu Thr Tyr Asp Arg Ala Asp Met Ala
        115                 120                 125

Phe Ala Asp Tyr Gly Leu Phe Trp Arg Gln Met Arg Lys Leu Cys Val
    130                 135                 140

Met Lys Leu Phe Ser Arg Lys Arg Ala Glu Ser Trp Asp Ser Val Arg
145                 150                 155                 160

Asp Glu Ala Asp Ser Met Val Arg Ile Val Thr Thr Asn Thr Gly Thr
                165                 170                 175

Ala Val Asn Leu Gly Glu Leu Val Phe Ser Leu Thr Arg Asn Ile Ile
            180                 185                 190

Tyr Arg Ala Ala Phe Gly Thr Cys Ser Glu Asp Gly Gln Gly Glu Phe
        195                 200                 205

Ile Lys Ile Met Gln Glu Phe Ser Lys Leu Phe Gly Ala Phe Asn Ile
    210                 215                 220

Ala Asp Phe Ile Pro Trp Leu Gly Trp Val Gly Lys Gln Ser Leu Asn
225                 230                 235                 240

Ile Arg Leu Ala Lys Ala Arg Ala Ser Leu Asp Gly Phe Ile Asp Ser
                245                 250                 255

Ile Ile Asp Asp His Ile Ile Arg Lys Lys Ala Tyr Val Asn Gly Lys
            260                 265                 270

Asn Asp Gly Gly Asp Arg Glu Thr Asp Met Val Asp Glu Leu Leu Ala
        275                 280                 285

Phe Tyr Ser Glu Glu Ala Lys Val Thr Glu Ser Glu Asp Leu Gln Asn
    290                 295                 300

Ala Ile Arg Leu Thr Lys Asp Asn Ile Lys Ala Ile Ile Met Asp Val
305                 310                 315                 320

Met Phe Gly Gly Thr Glu Thr Val Ala Ser Ala Ile Glu Trp Ala Met
                325                 330                 335

Ala Glu Leu Met Arg Ser Pro Glu Asp Leu Lys Lys Val Gln Gln Glu
            340                 345                 350

Leu Ala Asn Val Val Gly Leu Asn Arg Lys Val Glu Glu Ser Asp Phe
        355                 360                 365

Glu Lys Leu Thr Tyr Leu Arg Cys Cys Leu Lys Glu Thr Leu Arg Leu
    370                 375                 380

His Pro Pro Ile Pro Leu Leu Leu His Glu Thr Ala Glu Glu Ser Thr
385                 390                 395                 400

Val Ser Gly Tyr His Ile Pro Ala Lys Ser His Val Ile Ile Asn Ser
                405                 410                 415

Phe Ala Ile Gly Arg Asp Lys Asn Ser Trp Glu Asp Pro Glu Thr Tyr
            420                 425                 430

Lys Pro Ser Arg Phe Leu Lys Glu Gly Val Pro Asp Phe Lys Gly Gly
        435                 440                 445

Asn Phe Glu Phe Ile Pro Phe Gly Ser Gly Arg Arg Ser Cys Pro Gly
    450                 455                 460

Met Gln Leu Gly Leu Tyr Ala Leu Glu Met Ala Val Ala His Leu Leu
465                 470                 475                 480

His Cys Phe Thr Trp Glu Leu Pro Asp Gly Met Lys Pro Ser Glu Leu
                485                 490                 495

Lys Met Asp Asp Ile Phe Gly Leu Thr Ala Pro Arg Ala Asn Arg Leu
            500                 505                 510

Val Ala Val Pro Thr Pro Arg Leu Leu Cys Pro Leu Tyr
        515                 520                 525

<210>  17
<211>  1542
<212>  DNA
<213>  Populus trichocarpa

<400>  17
atggattctc ttctccaatc tttgcaaact ttacccatgt ctttcttctt gatcattatc      60

tcttcaattt tcttcttagg tctaatctct cgccttcgcc ggagatcacc atatccacca     120

gggcctaaag ggtttccact tattggtagc atgcatctga tggaccaatt aactgaccgt     180

ggcttagcta aactagctaa acaatatggt gggctatttc atatgcgtat ggggtacttg     240

catatggtag ctggttcatc tcctgaagta gctcgccaag ttttgcaagt ccaggataac     300

atgttctcca atagaccagc caacatagcc ataagctact taacctatga tcgtgccgat     360

atggcctttg cccactatgg tccgttctgg cgccagatgc gtaagctctg cgtcatgaag     420

ctatttagcc ggaaaagggc tgaatcatgg gagtctgtga gagacgaggt ggactcaatg     480

gttaagacag ttgaatccaa tataggcaag cctgtgaacg ttggagaatt gatatttaca     540

ctgaccatga atatcactta tagagcagct tttggggcca aaaatgaagg acaagatgag     600

ttcatcaaga ttttgcagga gttctccaag ctttttggag cattcaacat ttcggatttc     660

attccttggc ttggttggat tgacccccaa gggctcaccg ctagacttgt caaggctcga     720

aaagcacttg ataaattcat cgaccatatc atcgatgatc atatccaaaa aagaaaacag     780

aataactact ctgaagaggc tgaaaccgat atggttgatg acatgctaac cttttacagt     840

gaagagacaa aagtaaacga atcagatgat ttacaaaacg ccatcaaact tactagagac     900

aacatcaaag ccatcatcat ggatgtgatg tttggtggga cggagacagt ggcgtcggct     960

atagagtggg ccatggcaga gctattgaag agtccagaag atataaaaag agtccagcaa    1020

gagctcgccg acgtggttgg tttagagcgg cgcgtggagg aaagtgattt tgataaacta    1080

acattcttca aatgcacgct taaagaaacc ttaagacttc acccaccaat cccacttctc    1140

ttacacgaaa catctgaaga tgctgaggtt gctggttatt acgttccaaa aaagacaagg    1200

gtcatgatca atgcttatgc tattggcaga gacaagaatt catgggaaga tcctgattct 1260

tttaagcctt cgaggttttt ggaaccaggg gtgcctgatt tcaaagggaa tcacttcgaa 1320

tttattccgt tcgggtcagg tcggaggtct tgccctggca tgcaactggg gttatacgcc 1380

cttgatttgg ctgttgctca tttgcttcat tgttttactt gggagttgcc tgatggcatg 1440

aaaccaagtg aactcgacat gactgatatg tttggactca ctgcgccaag agcaactcga 1500

ctcgttgccg tcccgagaaa gcgcgtggtc tgtccacttt aa 1542

<210> 18
<211> 513
<212> PRT
<213> Populus trichocarpa

<400> 18

Met Asp Ser Leu Leu Gln Ser Leu Gln Thr Leu Pro Met Ser Phe Phe
1               5                   10                  15

Leu Ile Ile Ile Ser Ser Ile Phe Phe Leu Gly Leu Ile Ser Arg Leu
            20                  25                  30

Arg Arg Arg Ser Pro Tyr Pro Pro Gly Pro Lys Gly Phe Pro Leu Ile
        35                  40                  45

Gly Ser Met His Leu Met Asp Gln Leu Thr Asp Arg Gly Leu Ala Lys
    50                  55                  60

Leu Ala Lys Gln Tyr Gly Gly Leu Phe His Met Arg Met Gly Tyr Leu
65                  70                  75                  80

His Met Val Ala Gly Ser Ser Pro Glu Val Ala Arg Gln Val Leu Gln
                85                  90                  95

Val Gln Asp Asn Met Phe Ser Asn Arg Pro Ala Asn Ile Ala Ile Ser
            100                 105                 110

Tyr Leu Thr Tyr Asp Arg Ala Asp Met Ala Phe Ala His Tyr Gly Pro
        115                 120                 125

Phe Trp Arg Gln Met Arg Lys Leu Cys Val Met Lys Leu Phe Ser Arg
    130                 135                 140

Lys Arg Ala Glu Ser Trp Glu Ser Val Arg Asp Glu Val Asp Ser Met
145                 150                 155                 160

Val Lys Thr Val Glu Ser Asn Ile Gly Lys Pro Val Asn Val Gly Glu
                165                 170                 175

Leu Ile Phe Thr Leu Thr Met Asn Ile Thr Tyr Arg Ala Ala Phe Gly
            180                 185                 190

Ala Lys Asn Glu Gly Gln Asp Glu Phe Ile Lys Ile Leu Gln Glu Phe
        195                 200                 205

Ser Lys Leu Phe Gly Ala Phe Asn Ile Ser Asp Phe Ile Pro Trp Leu
    210                 215                 220

Gly Trp Ile Asp Pro Gln Gly Leu Thr Ala Arg Leu Val Lys Ala Arg
225                 230                 235                 240

Lys Ala Leu Asp Lys Phe Ile Asp His Ile Ile Asp Asp His Ile Gln
                245                 250                 255

Lys Arg Lys Gln Asn Asn Tyr Ser Glu Glu Ala Glu Thr Asp Met Val
            260                 265                 270

Asp Asp Met Leu Thr Phe Tyr Ser Glu Glu Thr Lys Val Asn Glu Ser
        275                 280                 285

Asp Asp Leu Gln Asn Ala Ile Lys Leu Thr Arg Asp Asn Ile Lys Ala
    290                 295                 300

Ile Ile Met Asp Val Met Phe Gly Gly Thr Glu Thr Val Ala Ser Ala
305                 310                 315                 320

Ile Glu Trp Ala Met Ala Glu Leu Leu Lys Ser Pro Glu Asp Ile Lys
                325                 330                 335

Arg Val Gln Gln Glu Leu Ala Asp Val Val Gly Leu Glu Arg Arg Val
            340                 345                 350

Glu Glu Ser Asp Phe Asp Lys Leu Thr Phe Phe Lys Cys Thr Leu Lys
        355                 360                 365

Glu Thr Leu Arg Leu His Pro Pro Ile Pro Leu Leu Leu His Glu Thr
    370                 375                 380

Ser Glu Asp Ala Glu Val Ala Gly Tyr Tyr Val Pro Lys Lys Thr Arg
385                 390                 395                 400

Val Met Ile Asn Ala Tyr Ala Ile Gly Arg Asp Lys Asn Ser Trp Glu
                405                 410                 415

Asp Pro Asp Ser Phe Lys Pro Ser Arg Phe Leu Glu Pro Gly Val Pro

420                 425                 430

Asp Phe Lys Gly Asn His Phe Glu Phe Ile Pro Phe Gly Ser Gly Arg
        435                 440                 445

Arg Ser Cys Pro Gly Met Gln Leu Gly Leu Tyr Ala Leu Asp Leu Ala
    450                 455                 460

Val Ala His Leu Leu His Cys Phe Thr Trp Glu Leu Pro Asp Gly Met
465                 470                 475                 480

Lys Pro Ser Glu Leu Asp Met Thr Asp Met Phe Gly Leu Thr Ala Pro
                485                 490                 495

Arg Ala Thr Arg Leu Val Ala Val Pro Arg Lys Arg Val Val Cys Pro
            500                 505                 510

Leu

<210>  19
<211>  1560
<212>  DNA
<213>  Centaurium erythraea

<400>  19
   atggatatca gagaaaatca atttcttcaa gctctagctg cagacccctt gttaatcctc      60
   ctcctcttca tcactcctct ccttttcttg ttccttcttt caagattccg cctcaaaccc     120
   tacccgccag gccccagagg ctggcctttg atcggaaaca tgaccatgat ggaccaatta     180
   actcaccgtg gtcttgcgaa actcgccgga aagtacggtg gaatcctcca cctccgtatg     240
   ggattcgttc acacggtggc ggtttccagc ccggatatag ctcgacaagt tcttcaagtt     300
   caggacaaca ttttctccaa ccggccggct actatcgcga taagttattt aacttacgat     360
   agagccgaca tggcgttcgc tgattacggc cctttttggc gtcagatgag gaagctttgt     420
   gttatgaagc tcttcagccg aaaaagggcc gaatcatggg actccgttcg agatgaggtg     480
   gaacggatga cccatatagt ggctacgaac atcggctcgc cagttaatat tggcgagatg     540
   gtgttcgggc tcactaagga tgttatttac cgggccgcgt ttggcacaag ctcacatgaa     600
   gggcaagatg agttcattaa gattttgcaa gagttttcga aattgtttgg tgcttttaat     660
   ctggctgatt ttataccttg gttaagttgg tttgatccac aagggttaaa caagagacta     720
   gaaaaagctc gtgcttcgct tgatgggttt attgactcaa tcattgatga tcacatgaat     780
   agaaagaaga tcaaaagtgg atcaaatgat gttgtggaga gtgacatggt ggatgaattg     840
   cttgtgtttt tcagtgatga agctaaggtt ttggagtcgg aggatttgca gaactctatt     900
   aagctcacca gagacaatat caaagccatc atcatggatg taatgtttgg tgggacagag 960
   acagtagcct ctgcaatcga gtgggccatg gcggaactta tgagaaatcc agacgacctc 1020
   aagcgtgtac aacaggagct ctccgacgtg gtcggcctcc accggaaagt ccaagaatcc 1080
   gacttcgaaa agctcaccta cttcaaatgc tgcctcaaag aaaccctccg cctccaccct 1140
   ccgatccctc tcctcctcca cgagaccgcc gaagcctccg agatcgccgg ctaccggatt 1200
   ccggcgaagt cacgtgtcat gatcaacgcc tgggcgatag gccgggacaa ggattcctgg 1260
   aaggacccgg acacattccg gccggcgagg tttcttgagg aaggggttcc ggattttaag 1320
   ggaagcaatt tcgagtttat tccattcggg tcgggtcgga ggtcttgccc cggaatgcaa 1380
   ctgggtcttt atgctttgga agtggcggtg gcacatcttc tgcattgttt cacgtgggaa 1440
   ttgccagatg ggatgaagcc gagtgagctg gacatgggtg acgtatttgg actcaccgcg 1500
   ccgagagcga gtcgactcgt tgccgtgccg agtccacgtt tactttgccc actttactga 1560

<210> 20
<211> 519
<212> PRT
<213> Centaurium erythraea

<400> 20

Met Asp Ile Arg Glu Asn Gln Phe Leu Gln Ala Leu Ala Ala Asp Pro
1               5                   10                  15

Leu Leu Ile Leu Leu Leu Phe Ile Thr Pro Leu Leu Phe Leu Phe Leu
            20                  25                  30

Leu Ser Arg Phe Arg Leu Lys Pro Tyr Pro Pro Gly Pro Arg Gly Trp
        35                  40                  45

Pro Leu Ile Gly Asn Met Thr Met Met Asp Gln Leu Thr His Arg Gly
    50                  55                  60

Leu Ala Lys Leu Ala Gly Lys Tyr Gly Gly Ile Leu His Leu Arg Met
65                  70                  75                  80

Gly Phe Val His Thr Val Ala Val Ser Ser Pro Asp Ile Ala Arg Gln
                85                  90                  95

Val Leu Gln Val Gln Asp Asn Ile Phe Ser Asn Arg Pro Ala Thr Ile
            100                 105                 110

Ala Ile Ser Tyr Leu Thr Tyr Asp Arg Ala Asp Met Ala Phe Ala Asp
        115                 120                 125

Tyr Gly Pro Phe Trp Arg Gln Met Arg Lys Leu Cys Val Met Lys Leu

130                 135                 140

Phe Ser Arg Lys Arg Ala Glu Ser Trp Asp Ser Val Arg Asp Glu Val
145                 150                 155                 160

Glu Arg Met Thr His Ile Val Ala Thr Asn Ile Gly Ser Pro Val Asn
                165                 170                 175

Ile Gly Glu Met Val Phe Gly Leu Thr Lys Asp Val Ile Tyr Arg Ala
            180                 185                 190

Ala Phe Gly Thr Ser Ser His Glu Gly Gln Asp Glu Phe Ile Lys Ile
        195                 200                 205

Leu Gln Glu Phe Ser Lys Leu Phe Gly Ala Phe Asn Leu Ala Asp Phe
    210                 215                 220

Ile Pro Trp Leu Ser Trp Phe Asp Pro Gln Gly Leu Asn Lys Arg Leu
225                 230                 235                 240

Glu Lys Ala Arg Ala Ser Leu Asp Gly Phe Ile Asp Ser Ile Ile Asp
                245                 250                 255

Asp His Met Asn Arg Lys Lys Ile Lys Ser Gly Ser Asn Asp Val Val
            260                 265                 270

Glu Ser Asp Met Val Asp Glu Leu Leu Val Phe Phe Ser Asp Glu Ala
        275                 280                 285

Lys Val Leu Glu Ser Glu Asp Leu Gln Asn Ser Ile Lys Leu Thr Arg
    290                 295                 300

Asp Asn Ile Lys Ala Ile Ile Met Asp Val Met Phe Gly Gly Thr Glu
305                 310                 315                 320

Thr Val Ala Ser Ala Ile Glu Trp Ala Met Ala Glu Leu Met Arg Asn
                325                 330                 335

Pro Asp Asp Leu Lys Arg Val Gln Gln Glu Leu Ser Asp Val Val Gly
            340                 345                 350

Leu His Arg Lys Val Gln Glu Ser Asp Phe Glu Lys Leu Thr Tyr Phe
        355                 360                 365

Lys Cys Cys Leu Lys Glu Thr Leu Arg Leu His Pro Pro Ile Pro Leu
    370                 375                 380

Leu Leu His Glu Thr Ala Glu Ala Ser Glu Ile Ala Gly Tyr Arg Ile
385                 390                 395                 400

Pro Ala Lys Ser Arg Val Met Ile Asn Ala Trp Ala Ile Gly Arg Asp
                405                 410                 415

Lys Asp Ser Trp Lys Asp Pro Asp Thr Phe Arg Pro Ala Arg Phe Leu
            420                 425                 430

Glu Glu Gly Val Pro Asp Phe Lys Gly Ser Asn Phe Glu Phe Ile Pro
        435                 440                 445

Phe Gly Ser Gly Arg Arg Ser Cys Pro Gly Met Gln Leu Gly Leu Tyr
    450                 455                 460

Ala Leu Glu Val Ala Val Ala His Leu Leu His Cys Phe Thr Trp Glu
465                 470                 475                 480

Leu Pro Asp Gly Met Lys Pro Ser Glu Leu Asp Met Gly Asp Val Phe
                485                 490                 495

Gly Leu Thr Ala Pro Arg Ala Ser Arg Leu Val Ala Val Pro Ser Pro
            500                 505                 510

Arg Leu Leu Cys Pro Leu Tyr
        515

<210>  21
<211>  2088
<212>  DNA
<213>  Oryza sativa

<400>  21
agctctacac caagagcgct tataaacata aaaaacatct caaacacaac ccaaaatagg      60

gccaaaattc tagaatatta tttttgtttt gaaaggaatt taagtttgtg tggtgtgtca     120

tccatggcgg acatggtgaa gttcaccatg gagtggcttc aggatcctct gagcctggcg     180

atcgtcgtca cggtggccgt cctaatcatg cggatgcagc ggcggcgcgc ggcgccgttc     240

ccgccggggc cgaagccgct gccgatcgtc ggcaacatgg cgatgatgga ccagctgacg     300

caccgcggcc tggcagcgct ggcgaaggag tacggcggcc tgatgcacct ccggctcggc     360

cggctccacg cgttcgccgt gtcgacgccg gagtacgcgc gcgaggtgct ccaggcgcag     420

gacggcgcgt tctcgaacag gccggcgacc acggcgatcg cctacctcac ctacgaccgc     480

gcggacatgg cgttcgcgca ctacgggccc ttctggcgcc agatgaggaa gctgtgcgtg     540

gtgaagctct tcagccggcg ccgcgccgag acgtggctcg ccgtgcgcga cgagtccgcg     600

gcgctcgtgc gcgccgtggc ggcgtcgcgc ggcgaggccg ccgtcaacct cggcgagctc     660

atcttcaacc tcaccaagaa cgtcatcttc cgcgccgcgt tcggcacgcg cgacggcgag 720

gggcacgacg agttcatcgc catcctccag gagttctcca agctgttcgg cgcgttcaac 780

atcggcgact tcatcccctg gctcagctgg gcggacacca acggcatcaa cgcccgcctc 840

gtcgcggcgc gcaccgcgct cgacaggttc atcgacaaga tcatcgacga gcacatggag 900

cgcggcaaga accccgacga cgccgacgcc gacatggtcg acgacatgct cgcgttcctc 960

gccgaggcca agccgcacgc cggcaaggcc gccgccgccg ccgccggcgc cggcgacggg 1020

gccgacgacc tgcagaacac gctccgcctc acccgcgaca acatcaaggc catcatcatg 1080

gacgtgatgt tcggggggac ggagacggtg gcgtcggcga tcgagtgggc gatggcggag 1140

atgatgcaca gccccgacga cctccgccgc gtgcaggagg agctcgccgc cgtggtgggc 1200

ctcggccggg acgtcgccga gtccgacctc gacaagctcc ccttcctccg ctgcgtcatc 1260

aaggagacgc tccgcctcca cccgcccatc cccatcctcc tccacgagac cgccgccgac 1320

tgcctcgtcg ccggctactc cgtccccagg ggctcccgcg tcatggtcaa cgtgtgggcc 1380

atcgcccgcg accgcgccgc ctggggcccc gacgccgacg cgttccgccc ctcgcggttc 1440

gccgccggcg ccgccgccga ggggctcgac ttcaggggcg gctgcttcga gttcctcccc 1500

ttcggctccg gccgccgctc gtgcccgggc atggcgctgg ggctctacgc gctcgagctc 1560

gccgtcgcgc ggctcgccca cggcttcaac tggtcgctcc ccgacgggat gaagccgtcc 1620

gagctcgaca tgtccgacat cttcggcctc accgcgccgc gcgccacccg cctctccgcc 1680

gtcgccacgc cccggctcac ctgccccttg tactgacggc ggccggcgac cgcggcggcg 1740

tacggcaaga atggtttgca ggtgatggtg ttaaaagttc agacgcgtgt ggcgccattg 1800

gaggagcttg gtgtggctag cttagcttcc tctgggcctt tttttttttg ctggtttaat 1860

tttctttttt ttttctcctt tgtaatttaa tccatgtctc cagaacaata agtggattag 1920

tagggtataa ttaagggtga cagtgtgata tatagactat aagatcaaag ttgtaaattt 1980

ggtgtccggt gtgcctgtcc acataagcac agcctctgaa ttttcttgta aaactttgtc 2040

atgaaaattg agaaagaaaa cacctcgctt tccacgcgta cgcttacc 2088

<210> 22
<211> 530
<212> PRT
<213> Oryza sativa

<400> 22

Met Ala Asp Met Val Lys Phe Thr Met Glu Trp Leu Gln Asp Pro Leu
1               5                   10                  15

Ser Leu Ala Ile Val Val Thr Val Ala Val Leu Ile Met Arg Met Gln
            20                  25                  30

Arg Arg Arg Ala Ala Pro Phe Pro Pro Gly Pro Lys Pro Leu Pro Ile
        35                  40                  45

Val Gly Asn Met Ala Met Met Asp Gln Leu Thr His Arg Gly Leu Ala
    50                  55                  60

Ala Leu Ala Lys Glu Tyr Gly Gly Leu Met His Leu Arg Leu Gly Arg
65                  70                  75                  80

Leu His Ala Phe Ala Val Ser Thr Pro Glu Tyr Ala Arg Glu Val Leu
                85                  90                  95

Gln Ala Gln Asp Gly Ala Phe Ser Asn Arg Pro Ala Thr Thr Ala Ile
            100                 105                 110

Ala Tyr Leu Thr Tyr Asp Arg Ala Asp Met Ala Phe Ala His Tyr Gly
        115                 120                 125

Pro Phe Trp Arg Gln Met Arg Lys Leu Cys Val Val Lys Leu Phe Ser
    130                 135                 140

Arg Arg Arg Ala Glu Thr Trp Leu Ala Val Arg Asp Glu Ser Ala Ala
145                 150                 155                 160

Leu Val Arg Ala Val Ala Ala Ser Arg Gly Glu Ala Ala Val Asn Leu
                165                 170                 175

Gly Glu Leu Ile Phe Asn Leu Thr Lys Asn Val Ile Phe Arg Ala Ala
            180                 185                 190

Phe Gly Thr Arg Asp Gly Glu Gly His Asp Glu Phe Ile Ala Ile Leu
        195                 200                 205

Gln Glu Phe Ser Lys Leu Phe Gly Ala Phe Asn Ile Gly Asp Phe Ile
    210                 215                 220

Pro Trp Leu Ser Trp Ala Asp Thr Asn Gly Ile Asn Ala Arg Leu Val
225                 230                 235                 240

Ala Ala Arg Thr Ala Leu Asp Arg Phe Ile Asp Lys Ile Ile Asp Glu
                245                 250                 255

His Met Glu Arg Gly Lys Asn Pro Asp Asp Ala Asp Ala Asp Met Val
            260                 265                 270

Asp Asp Met Leu Ala Phe Leu Ala Glu Ala Lys Pro His Ala Gly Lys

275                 280                 285

Ala Ala Ala Ala Ala Ala Gly Ala Gly Asp Gly Ala Asp Asp Leu Gln
    290                 295                 300

Asn Thr Leu Arg Leu Thr Arg Asp Asn Ile Lys Ala Ile Ile Met Asp
305                 310                 315                 320

Val Met Phe Gly Gly Thr Glu Thr Val Ala Ser Ala Ile Glu Trp Ala
                325                 330                 335

Met Ala Glu Met Met His Ser Pro Asp Asp Leu Arg Arg Val Gln Glu
            340                 345                 350

Glu Leu Ala Ala Val Val Gly Leu Gly Arg Asp Val Ala Glu Ser Asp
        355                 360                 365

Leu Asp Lys Leu Pro Phe Leu Arg Cys Val Ile Lys Glu Thr Leu Arg
    370                 375                 380

Leu His Pro Pro Ile Pro Ile Leu Leu His Glu Thr Ala Ala Asp Cys
385                 390                 395                 400

Leu Val Ala Gly Tyr Ser Val Pro Arg Gly Ser Arg Val Met Val Asn
                405                 410                 415

Val Trp Ala Ile Ala Arg Asp Arg Ala Ala Trp Gly Pro Asp Ala Asp
            420                 425                 430

Ala Phe Arg Pro Ser Arg Phe Ala Ala Gly Ala Ala Ala Glu Gly Leu
        435                 440                 445

Asp Phe Arg Gly Gly Cys Phe Glu Phe Leu Pro Phe Gly Ser Gly Arg
    450                 455                 460

Arg Ser Cys Pro Gly Met Ala Leu Gly Leu Tyr Ala Leu Glu Leu Ala
465                 470                 475                 480

Val Ala Arg Leu Ala His Gly Phe Asn Trp Ser Leu Pro Asp Gly Met
                485                 490                 495

Lys Pro Ser Glu Leu Asp Met Ser Asp Ile Phe Gly Leu Thr Ala Pro
            500                 505                 510

Arg Ala Thr Arg Leu Ser Ala Val Ala Thr Pro Arg Leu Thr Cys Pro
        515                 520                 525

Leu Tyr
    530

<210>  23
<211>  1599
<212>  DNA
<213>  Zea mays

<400>  23
atggcggcgg ccgtggccaa tatcggcatg gagtggctcc aagaccctct gagctgggtg      60

ttcctgggca cggtgtgctt ggtggtcctg cagcagctgc gacgtcggcg gggcaaagcg     120

ccgcttccgc ctgggccgaa gccgctgccg atcgtaggca acatggggat gatggaccag     180

ctgacgcacc gcgggctggc ggcgctggcg gagacgtacg gcgggctgct gcacctccgg     240

ctggggcggc tgcacgcgtt cgcggtgtcg acgcccgagt acgcgcgcga ggtgctgcag     300

gcgcaggacg gcgcgttctc gaaccggcct gcgaccgcgg ccatcgcgta cctgacgtac     360

gaccgcgcgg acatggcgtt cgcgcactac gggcccttct ggcggcagat gcgcaagctg     420

tgcgtgatga agctgttcag ccggcggcgc gccgagacgt gggcggccgt gcgcgacgag     480

tgcgcggcgc tggtccgggc cgtggccgtg ggcggcggga gcgggggcga ggccgtgaac     540

ctgggcgagc tcatcttcag cctgacgaag aacgtgacgt tccgcgcggc gttcggcacc     600

cgcgacggcg agggccagga ggagttcatc gccatcctgc aggagttctc caagctgttc     660

ggcgccttca acgtgggcga cttcctaccg tggctgggct ggatggacct gcagggcatc     720

aaccggcgcc tgcgcgccgc ccgctccgcg ctggaccggt tcatcgacaa gatcatcgac     780

gagcacgtga ggcgcgggaa gagccccgac gacgccgacg ccgacatggt cgacgacatg     840

ctcgcgttct tcgtcgaggc cacgcccggc aaggcgacgg gcgccgccgc cgctgctgac     900

ggcggcgacg acctgcacaa caccctccgg ctcacgcgcg acaacatcaa ggccatcatc     960

atggacgtga tgttcggcgg gacggagacg gtggcgtcgg cgatcgagtg ggccatggcg    1020

gagatgatgc acagccccga cgacctgcgc cgggtgcagc aggagctcgc cgacgtcgtg    1080

ggcctcgacc gcaacgtgag cgagtcggac ctggacaggc tccccttcct caggtgcgtc    1140

atcaaggaga cgctccggct gcacccgccc atcccgctgc tcctccacga gaccgccgac    1200

gactgcgtcg tggccgggta ctccgtgccc aggggctccc gcgtcatggt caacgtctgg    1260

gccatcggcc gccaccgcgc ctcgtggaag gacgccgacg cgttccgccc gtcgcggttc    1320

gcggcgcccg agggggaggc cgcggggctc gacttcaagg gcgggtgctt cgagttcctg    1380

ccgttcgggt cgggccgccg gtcctgcccc gggatggcgc tcggcctgta cgcgctggag    1440

ctcgccgtcg cccagctcgc gcacgccttc aactggtcgc tgcccgacgg aatgaagccc    1500

tcggagatgg acatgggcga catcttcggc cttaccgcgc cgcgcgccac gcggctctac    1560

gccgtgccta cgccccggct caactgcccc ttgtactga                           1599

<210>  24
<211>  532
<212>  PRT
<213>  Zea mays

<400>  24

Met Ala Ala Ala Val Ala Asn Ile Gly Met Glu Trp Leu Gln Asp Pro
1               5                   10                  15

Leu Ser Trp Val Phe Leu Gly Thr Val Cys Leu Val Val Leu Gln Gln
            20                  25                  30

Leu Arg Arg Arg Arg Gly Lys Ala Pro Leu Pro Pro Gly Pro Lys Pro
        35                  40                  45

Leu Pro Ile Val Gly Asn Met Gly Met Met Asp Gln Leu Thr His Arg
    50                  55                  60

Gly Leu Ala Ala Leu Ala Glu Thr Tyr Gly Gly Leu Leu His Leu Arg
65                  70                  75                  80

Leu Gly Arg Leu His Ala Phe Ala Val Ser Thr Pro Glu Tyr Ala Arg
                85                  90                  95

Glu Val Leu Gln Ala Gln Asp Gly Ala Phe Ser Asn Arg Pro Ala Thr
            100                 105                 110

Ala Ala Ile Ala Tyr Leu Thr Tyr Asp Arg Ala Asp Met Ala Phe Ala
        115                 120                 125

His Tyr Gly Pro Phe Trp Arg Gln Met Arg Lys Leu Cys Val Met Lys
    130                 135                 140

Leu Phe Ser Arg Arg Arg Ala Glu Thr Trp Ala Ala Val Arg Asp Glu
145                 150                 155                 160

Cys Ala Ala Leu Val Arg Ala Val Ala Val Gly Gly Gly Ser Gly Gly
                165                 170                 175

Glu Ala Val Asn Leu Gly Glu Leu Ile Phe Ser Leu Thr Lys Asn Val
            180                 185                 190

Thr Phe Arg Ala Ala Phe Gly Thr Arg Asp Gly Glu Gly Gln Glu Glu
        195                 200                 205

Phe Ile Ala Ile Leu Gln Glu Phe Ser Lys Leu Phe Gly Ala Phe Asn
    210                 215                 220

Val Gly Asp Phe Leu Pro Trp Leu Gly Trp Met Asp Leu Gln Gly Ile
225                 230                 235                 240

Asn Arg Arg Leu Arg Ala Ala Arg Ser Ala Leu Asp Arg Phe Ile Asp
                245                 250                 255

Lys Ile Ile Asp Glu His Val Arg Arg Gly Lys Ser Pro Asp Asp Ala
            260                 265                 270

Asp Ala Asp Met Val Asp Asp Met Leu Ala Phe Phe Val Glu Ala Thr
        275                 280                 285

Pro Gly Lys Ala Thr Gly Ala Ala Ala Ala Ala Asp Gly Gly Asp Asp
    290                 295                 300

Leu His Asn Thr Leu Arg Leu Thr Arg Asp Asn Ile Lys Ala Ile Ile
305                 310                 315                 320

Met Asp Val Met Phe Gly Gly Thr Glu Thr Val Ala Ser Ala Ile Glu
                325                 330                 335

Trp Ala Met Ala Glu Met Met His Ser Pro Asp Asp Leu Arg Arg Val
            340                 345                 350

Gln Gln Glu Leu Ala Asp Val Val Gly Leu Asp Arg Asn Val Ser Glu
        355                 360                 365

Ser Asp Leu Asp Arg Leu Pro Phe Leu Arg Cys Val Ile Lys Glu Thr
    370                 375                 380

Leu Arg Leu His Pro Pro Ile Pro Leu Leu Leu His Glu Thr Ala Asp
385                 390                 395                 400

Asp Cys Val Val Ala Gly Tyr Ser Val Pro Arg Gly Ser Arg Val Met
                405                 410                 415

Val Asn Val Trp Ala Ile Gly Arg His Arg Ala Ser Trp Lys Asp Ala
            420                 425                 430

Asp Ala Phe Arg Pro Ser Arg Phe Ala Ala Pro Glu Gly Glu Ala Ala
        435                 440                 445

Gly Leu Asp Phe Lys Gly Gly Cys Phe Glu Phe Leu Pro Phe Gly Ser
    450                 455                 460

Gly Arg Arg Ser Cys Pro Gly Met Ala Leu Gly Leu Tyr Ala Leu Glu

465                 470                 475                 480

Leu Ala Val Ala Gln Leu Ala His Ala Phe Asn Trp Ser Leu Pro Asp
                485                 490                 495

Gly Met Lys Pro Ser Glu Met Asp Met Gly Asp Ile Phe Gly Leu Thr
            500                 505                 510

Ala Pro Arg Ala Thr Arg Leu Tyr Ala Val Pro Thr Pro Arg Leu Asn
        515                 520                 525

Cys Pro Leu Tyr
    530

<210>  25
<211>  2202
<212>  DNA
<213>  Broussonetia papyrifera

<400>  25
gcaaatatgg ataccaaaag tatcaccctc ctacaagagg ccctcaagcc cttgcccatg      60

gccatcttct tagtcatccc cctcctcttc ctcctcggcc atatcctccg gtcccggggg     120

aggctgccgt atccgccggg cccgaagggc cttccgatca tcgggagcat gtcgatgatg     180

gaccagctga cccaccgcgg cctcgccgcc ctcgccaagc agtacggcgg catcttccac     240

ctcaagatgg ggtacctcca catggtggcg atctcgtcgc cggaaacggc ccggcaggtc     300

ctccaactcc aggacaacat cttctcgaac cggccagcga atatcgccat caggtacctg     360

acgtacgacc gggcggacat ggcgttcgcc cactacggcc cgttctggcg gcagatgcgg     420

aagctgtgcg tgatgaagct cttcagccgc aagcgggcgg cgtcgtggga gtcggtgcgc     480

ggggaggtgg agaagacggt cctctccgtg gcgggcagca ccgggacggc ggtgaacgtg     540

ggagagctgg tgttcaagac gacgatggat ataatctaca gagcggcgtt cggggcgagc     600

aacaaggaag ggcaggacga gttcataagc atattgcagg agttttcgaa gctgttcggg     660

gccttcaaca tggcggactt cattccgtgg ctgacttggg tggatccgca ggggctgaac     720

ggcaggctga agagggcgcg gaaggcgttg gatgcgttta tagacaagat aatagacgag     780

cacgtggtga agaagaatga ggcggccgag accgacatgg tcgatgatct tctggccttt     840

tacagtaacg aggaggccaa ggtgaatgac gcggaggatc tgcacaacgc cattaggctc     900

actagagata atatcaaggc catcatcatg gtaagtacta agtaattagc ttgtcccaat     960

cattttgtgt gtgtgtgtgt ttttttaaat cgaagataaa gaattcaaac tcttatttat    1020

tttatggtag agtcgcatgt atctgcaatc acataaataa ataataataa tcagccgaat    1080

tggtggacgt tacacaaaaa gggaaaaaga aagaaaagag tgagcattca aatttggaaa    1140

attagtcgtg aaatgaattt tcaatatgta tctttccact attgaccatt atgaaagata 1200

tttttaaaaa taaccagtaa gaaagtagga aattagttgg gatatgaaaa ttgaaggaga 1260

gtctaatgaa aaatgatttt ttatagttga aaaatattaa aaagttgact aagtgttaag 1320

gaaaactaaa taaataatat gtatataaac acgccattta acgttattcg caaccgaaaa 1380

ataaaatgta acgttacata tgcaaagcat gatatttgtt taaacaattt taattcgtga 1440

tcgtatacgt attagaaagt taatagaatt tcaggaaaat cttagcagat tttgatagga 1500

tattcaatta tgcttttttt tttatcactc aaaaaaaaaa agggtcaggg cattaacgat 1560

atggcgtgta aatatcacag gacgtgatgt tcggcgggac ggagacagtg gcatcggcta 1620

ttgagtgggc catgacggag atgatgaggt gcccagaaga tttgaagaag gtccaacaag 1680

agctggccga tgtggtgggc ctggatcgga ggctggaaga gtccgacttc gagaagctga 1740

cctacctccg gtgcgccatc aaggagaccc tccggctcca ccctccgatc cccctcctcc 1800

tccacgagac ggcggaggac gccgccgtgg ccgggtacca catccccaag ggatcacgtg 1860

taatgatcaa tgcatgggcc atagggaggg acaagaactc gtgggccgac ccggacagtt 1920

ttaagcccgc taggtttttg agggatgggg tgcctgattt caaaggaagt aactttgagt 1980

tcatcccgtt tgggtcgggt cggaggtcct gccccgggat gcaactcggg ctttacgcgc 2040

tcgagctgac agtgggccac ctcctccact gcttcacgtg gaatttgccc gatgggatga 2100

agccaagtga gctcgacatg aatgatgtgt tcgggcttac cgcaccgagg gcgacccgac 2160

tcgttgcgat cccgactaaa cgtgtcgtgt gcaccatctg at 2202

<210> 26
<211> 514
<212> PRT
<213> Broussonetia papyrifera

<400> 26

Met Asp Thr Lys Ser Ile Thr Leu Leu Gln Glu Ala Leu Lys Pro Leu
1               5                   10                  15

Pro Met Ala Ile Phe Leu Val Ile Pro Leu Leu Phe Leu Leu Gly His
            20                  25                  30

Ile Leu Arg Ser Arg Gly Arg Leu Pro Tyr Pro Pro Gly Pro Lys Gly
        35                  40                  45

Leu Pro Ile Ile Gly Ser Met Ser Met Met Asp Gln Leu Thr His Arg
    50                  55                  60

Gly Leu Ala Ala Leu Ala Lys Gln Tyr Gly Gly Ile Phe His Leu Lys
65                  70                  75                  80

Met Gly Tyr Leu His Met Val Ala Ile Ser Ser Pro Glu Thr Ala Arg
                85                  90                  95

Gln Val Leu Gln Leu Gln Asp Asn Ile Phe Ser Asn Arg Pro Ala Asn
            100                 105                 110

Ile Ala Ile Arg Tyr Leu Thr Tyr Asp Arg Ala Asp Met Ala Phe Ala
        115                 120                 125

His Tyr Gly Pro Phe Trp Arg Gln Met Arg Lys Leu Cys Val Met Lys
    130                 135                 140

Leu Phe Ser Arg Lys Arg Ala Ala Ser Trp Glu Ser Val Arg Gly Glu
145                 150                 155                 160

Val Glu Lys Thr Val Leu Ser Val Ala Gly Ser Thr Gly Thr Ala Val
                165                 170                 175

Asn Val Gly Glu Leu Val Phe Lys Thr Thr Met Asp Ile Ile Tyr Arg
            180                 185                 190

Ala Ala Phe Gly Ala Ser Asn Lys Glu Gly Gln Asp Glu Phe Ile Ser
        195                 200                 205

Ile Leu Gln Glu Phe Ser Lys Leu Phe Gly Ala Phe Asn Met Ala Asp
    210                 215                 220

Phe Ile Pro Trp Leu Thr Trp Val Asp Pro Gln Gly Leu Asn Gly Arg
225                 230                 235                 240

Leu Lys Arg Ala Arg Lys Ala Leu Asp Ala Phe Ile Asp Lys Ile Ile
                245                 250                 255

Asp Glu His Val Val Lys Lys Asn Glu Ala Ala Glu Thr Asp Met Val
            260                 265                 270

Asp Asp Leu Leu Ala Phe Tyr Ser Asn Glu Glu Ala Lys Val Asn Asp
        275                 280                 285

Ala Glu Asp Leu His Asn Ala Ile Arg Leu Thr Arg Asp Asn Ile Lys
    290                 295                 300

Ala Ile Ile Met Asp Val Met Phe Gly Gly Thr Glu Thr Val Ala Ser
305                 310                 315                 320

Ala Ile Glu Trp Ala Met Thr Glu Met Met Arg Cys Pro Glu Asp Leu
                325                 330                 335

Lys Lys Val Gln Gln Glu Leu Ala Asp Val Val Gly Leu Asp Arg Arg
            340                 345                 350

Leu Glu Glu Ser Asp Phe Glu Lys Leu Thr Tyr Leu Arg Cys Ala Ile
        355                 360                 365

Lys Glu Thr Leu Arg Leu His Pro Pro Ile Pro Leu Leu Leu His Glu
    370                 375                 380

Thr Ala Glu Asp Ala Ala Val Ala Gly Tyr His Ile Pro Lys Gly Ser
385                 390                 395                 400

Arg Val Met Ile Asn Ala Trp Ala Ile Gly Arg Asp Lys Asn Ser Trp
                405                 410                 415

Ala Asp Pro Asp Ser Phe Lys Pro Ala Arg Phe Leu Arg Asp Gly Val
            420                 425                 430

Pro Asp Phe Lys Gly Ser Asn Phe Glu Phe Ile Pro Phe Gly Ser Gly
        435                 440                 445

Arg Arg Ser Cys Pro Gly Met Gln Leu Gly Leu Tyr Ala Leu Glu Leu
    450                 455                 460

Thr Val Gly His Leu Leu His Cys Phe Thr Trp Asn Leu Pro Asp Gly
465                 470                 475                 480

Met Lys Pro Ser Glu Leu Asp Met Asn Asp Val Phe Gly Leu Thr Ala
                485                 490                 495

Pro Arg Ala Thr Arg Leu Val Ala Ile Pro Thr Lys Arg Val Val Cys
            500                 505                 510

Thr Ile

<210>  27
<211>  1569
<212>  DNA
<213>  Solanum tuberosum

<400>  27
atgaaagaaa tggtactaaa caatatcaat tcaactcttg aagctttaca agctcagccc      60

atgctactct tcttcttcat catccctctc ttcttcttat accttttttc gacgtctcgc     120

cggaaacgtt atcctccagg accattaggc tggcctctca ttggtaacat gatgatgatg     180

gaccagttaa ctcaccgtgg tcttgccaaa ctagcacaaa aatatggtgg cgtatttcac     240

ctcaaaatgg gttatgtcca caaaattgtt atatctggtc cagaggaagc tcgtcaagta 300

ctacaggtac aagacaacat atattcgaac cgtccaaaga ccgtagccat aagttaccta 360

acgtacgatc gtgcggacat ggcttttgct gactacggac ccttttggcg tcagatgaga 420

aaattatgtg ttatgaagct attcagtcgt aaacgagctg agtcatggga ctcagttcgc 480

gacgaagtgg actcaatggt aaagattgtt acaaccaaca cgggtacatc cattaactta 540

ggggaacttg ttttctgtct cactcgtaac attatctacc gagcagcttt cggaacaagt 600

tcagatgaag gacaagatga ttttattaaa attttgcaag aattttcgaa gctatttggt 660

gcgtttaaca tggctgattt tattccatgg ctaaggtgga ttggtcagca aggtctaaat 720

gttagacttg ctaaggcaag ggcatcactt gatggattta ttgattcgat tattgatgat 780

catattgaaa gaaagaaggc taatgttatt aatgatgatg gttatagaga aagtgatatg 840

gtcgatgagc ttttagcttt ttacagtgaa gaaacaaaag taaatgagtc tgaagatttg 900

cagaattcta taaggcttac tagggataat atcaaagcta taatcatgga tgtaatgttt 960

ggtgggacag agacagtggc atctgcgata gaatgggcaa tggcagagct tatgaaaagt 1020

ccagaggatc ttaaaaaggt acaacaagaa ttagctaacg ttgttggact gaataggaaa 1080

gttgacgaat ccgattttga aaaattgacc tacttaaaat gttgtttaaa agaaactctc 1140

cgacttcacc ctcctatccc tctcctcctc cacgaaaccg cggaggaatc gacagtctct 1200

ggctactata ttccagcaaa atcacatgtt atcataaatt catttgcaat tggacgtgac 1260

aaaaactcat gggaagatcc tgatagtttc aagcctagta ggttccttaa agaaggtgtg 1320

ccagatttta aaggtggtaa ttttgaattt ttaccatttg ggtcgggtcg aaggtcttgc 1380

cccggtatgc aacttgggct ttatgcatta gaaatggctg tggcccattt acttcattgt 1440

tttacttggg aattacctga tgggatgaaa ccaagtgagc taaaaatgga tgatatattt 1500

gggcttactg ctccattggc taatcgacta gtggctgtgc ctactccacg tttattatgt 1560

aattattga 1569

<210> 28
<211> 522
<212> PRT
<213> Solanum tuberosum

<400> 28

Met Lys Glu Met Val Leu Asn Asn Ile Asn Ser Thr Leu Glu Ala Leu
1               5                   10                  15

Gln Ala Gln Pro Met Leu Leu Phe Phe Phe Ile Ile Pro Leu Phe Phe
            20                  25                  30

Leu Tyr Leu Phe Ser Thr Ser Arg Arg Lys Arg Tyr Pro Pro Gly Pro
        35                  40                  45

Leu Gly Trp Pro Leu Ile Gly Asn Met Met Met Met Asp Gln Leu Thr
    50                  55                  60

His Arg Gly Leu Ala Lys Leu Ala Gln Lys Tyr Gly Gly Val Phe His
65                  70                  75                  80

Leu Lys Met Gly Tyr Val His Lys Ile Val Ile Ser Gly Pro Glu Glu
                85                  90                  95

Ala Arg Gln Val Leu Gln Val Gln Asp Asn Ile Tyr Ser Asn Arg Pro
            100                 105                 110

Lys Thr Val Ala Ile Ser Tyr Leu Thr Tyr Asp Arg Ala Asp Met Ala
        115                 120                 125

Phe Ala Asp Tyr Gly Pro Phe Trp Arg Gln Met Arg Lys Leu Cys Val
    130                 135                 140

Met Lys Leu Phe Ser Arg Lys Arg Ala Glu Ser Trp Asp Ser Val Arg
145                 150                 155                 160

Asp Glu Val Asp Ser Met Val Lys Ile Val Thr Thr Asn Thr Gly Thr
                165                 170                 175

Ser Ile Asn Leu Gly Glu Leu Val Phe Cys Leu Thr Arg Asn Ile Ile
            180                 185                 190

Tyr Arg Ala Ala Phe Gly Thr Ser Ser Asp Glu Gly Gln Asp Asp Phe
        195                 200                 205

Ile Lys Ile Leu Gln Glu Phe Ser Lys Leu Phe Gly Ala Phe Asn Met
    210                 215                 220

Ala Asp Phe Ile Pro Trp Leu Arg Trp Ile Gly Gln Gln Gly Leu Asn
225                 230                 235                 240

Val Arg Leu Ala Lys Ala Arg Ala Ser Leu Asp Gly Phe Ile Asp Ser
                245                 250                 255

Ile Ile Asp Asp His Ile Glu Arg Lys Lys Ala Asn Val Ile Asn Asp
            260                 265                 270

Asp Gly Tyr Arg Glu Ser Asp Met Val Asp Glu Leu Leu Ala Phe Tyr
        275                 280                 285

Ser Glu Glu Thr Lys Val Asn Glu Ser Glu Asp Leu Gln Asn Ser Ile
    290                 295                 300

Arg Leu Thr Arg Asp Asn Ile Lys Ala Ile Ile Met Asp Val Met Phe
305                 310                 315                 320

Gly Gly Thr Glu Thr Val Ala Ser Ala Ile Glu Trp Ala Met Ala Glu
                325                 330                 335

Leu Met Lys Ser Pro Glu Asp Leu Lys Lys Val Gln Gln Glu Leu Ala
            340                 345                 350

Asn Val Val Gly Leu Asn Arg Lys Val Asp Glu Ser Asp Phe Glu Lys
        355                 360                 365

Leu Thr Tyr Leu Lys Cys Cys Leu Lys Glu Thr Leu Arg Leu His Pro
    370                 375                 380

Pro Ile Pro Leu Leu Leu His Glu Thr Ala Glu Glu Ser Thr Val Ser
385                 390                 395                 400

Gly Tyr Tyr Ile Pro Ala Lys Ser His Val Ile Ile Asn Ser Phe Ala
                405                 410                 415

Ile Gly Arg Asp Lys Asn Ser Trp Glu Asp Pro Asp Ser Phe Lys Pro
            420                 425                 430

Ser Arg Phe Leu Lys Glu Gly Val Pro Asp Phe Lys Gly Gly Asn Phe
        435                 440                 445

Glu Phe Leu Pro Phe Gly Ser Gly Arg Arg Ser Cys Pro Gly Met Gln
    450                 455                 460

Leu Gly Leu Tyr Ala Leu Glu Met Ala Val Ala His Leu Leu His Cys
465                 470                 475                 480

Phe Thr Trp Glu Leu Pro Asp Gly Met Lys Pro Ser Glu Leu Lys Met
                485                 490                 495

Asp Asp Ile Phe Gly Leu Thr Ala Pro Leu Ala Asn Arg Leu Val Ala
            500                 505                 510

Val Pro Thr Pro Arg Leu Leu Cys Asn Tyr
        515                 520

<210>  29
<211>  2648

<212>  DNA
<213>  Arabidopsis thaliana

<400>  29
agtgtaagaa caatgagtca atgactacgt gcaggacatt ggacataccg tgtgttcttt      60

tggattgaaa tgttgtttcg aagggctgtt agttgatgtt gaaaataggt tgaagttgaa     120

taatgcatgt tgatatagta aatatcaatg gtaatatttt ctcatttccc aaaactcaaa     180

tgatatcatt taactataaa ctaacgtaaa ctgttgacaa tacacttatg gttaaaaatt     240

tggagtcttg ttttagtata cgtatcacca ccgcacggtt tcaaaaccac ataattgtaa     300

atgttattgg aaaatagaac tcgcaatacg tattgtattt tggtaaacat agctctaagc     360

ctctaatata taagctctca acaattctgg ctaatggtcc caagtaagaa aagcccatgt     420

attgtaaggt catgatctca aaaacgaggg tgaggtggaa tactaacatg aggagaaagt     480

aaggtgacaa atttttgggg caatagtggt ggatatggtg gggaggtagg tagcatcatt     540

tctccaagtc gctgtctttc gtggtaatgg taggtgtgtc tctctttata ttatttatta     600

ctactcattg taaatttctt ttttctacaa tttgtttctg actccaaaat acgtcacaaa     660

tataatacta ggcaaataat tattttatta taagtcaata gagtggttgt tgtaaaattg     720

attttttgat attgaaagag ttcatggacg gatgtgtatg cgccaaatgg taagcccttg     780

tactgtgccg cgcgtatatt ttaaccacca ctagttgttt ctctttttca aaaaacacaa     840

aaaaaaaata atttgttttc ttaacggcgt caaatctgac ggcgtctcaa tacgttcaat     900

ttttttcttt ctttcacatg gtttctcata gctttgcatt gaccataggt aaagggataa     960

ggataatggt tttttctctt gtttgtttta tccttattat tcaaaaagga taaaaaaaca    1020

gtgatattta gatttctttg attaaaaaag tcattgaaat tcatatttga ttttttgcta    1080

aatgtcaaca cagagacaca aacgtaatgc actgtcgcca atattcatgg atcatgacaa    1140

taaatatcac tagaataatt aaaaatcagt agaatgcaaa caaagcattt tctaagtaaa    1200

acagtctttt atattcacgt aattggaatt tccttttttt ttttttgtcg taattggaat    1260

ttcctttatc aaacccaaag tccaaaacaa tcggcaatgt tttgcaaaat gttcaaaact    1320

attggcgggt tggtctatcc gaattgaaga tcttttctcc atatgataga ccaacgaaat    1380

tcggcatacg tgtttttttt tttgttttga aaacccttta aacaacctta attcaaaata    1440

ctaatgtaac tttattgaac gtgcatctaa aaattttgaa ctttgctttt gagaaataat    1500

caatgtacca ataaagaaga tgtagtacat acattataat taaatacaaa aaaggaatca    1560

ccatatagta catggtagac aatgaaaaac tttaaaacat atacaatcaa taatactctt    1620

tgtgcataac tttttttgtc gtctcgagtt tatatttgag tacttataca aactattaga    1680

ttacaaactg tgctcagata cattaagtta atcttatata caagagcact cgagtgttgt    1740

ccttaagtta atcttaagat atcttgaggt aaatagaaat agttgactcg tttttatctt    1800

cttctttttt taccatgagc aaaaaagatg aaataagttc aaaacgtgac gaatctatat 1860

gttactactt agtatgtgtc aatcattaaa tcgggaaaac ttcatcattt caggagtatt 1920

acaaaactcc taagagtgag aacgactaca tagtacatat tttgataaaa gacttgaaaa 1980

cttgctaaaa cgaatttgcg aaaatataat catacaagtg ccagtgattt tgatcgaatt 2040

attcatagct ttgtaggatg aacttaatta aataatatct cacaaaagta ttgacagtaa 2100

cctagtacta tactatctat gttagaatat gattatgata taatttatcc cctcacttat 2160

tcatatgatt tttgaagcaa ctactttcgt ttttttaaca ttttcttttg ttggttattg 2220

ttaatgagca tatttagtcg tttcttaatt ccactgaaat agaaaataca aagagaactt 2280

tagttaatag atatgaacat aatctcacat cctcctccta ccttcaccaa acacttttac 2340

atacactttg tggtctttct ttacctacca ccatcaacaa caacaccaag ccccactcac 2400

acacacgcaa tcacgttaaa tttaacgccg tttattatct catcattcac caactcccac 2460

gtacctaacg ccgtttacct tttgccgttg gtcctcattt ctcaaaccaa ccaaacctct 2520

ccctcttata aaatcctctc tcccttcttt atttcttcct cagcagcttc ttctgctttc 2580

aattactctc gccgacgatt ttctcaccgg aaaaaaacaa tatcattgcg gatacacaaa 2640

ctataatg 2648

<210> 30
<211> 2869
<212> DNA
<213> Arabidopsis thaliana

<400> 30
tagatgggtt ttcaattttt tttttacttt ttgttagaag caaaattggg gagaagagta 60

acttcttttt ttgatacttg taagtttgaa ttccttttcc cgtgcttcaa aagttttata 120

ttttatttat agttttgatt tcttattgta actaagggat tcgattttga tcgttgacta 180

gctatacaat cccgtgtaat cactacattt ggtattggta cgcatctgtt acataccatt 240

tttacggcgg ttttagtgaa ctatattata aaaaaaaatc gtaagttttt ttgtgtgtgt 300

gttaacaatg tactcactac tcactgttcc atatttttga tgtacgtata tcgaaaacat 360

tctgccaaca aatgcaaaca taacaaaagt caaaaacaat aacataaccg ggaattaaac 420

caaaatgtaa ttgcttttta ttagtgtcag gccttctgct taaaaatatt ctcggcccag 480

agcccattaa cacctatctc aattcatatt gaagaaaatg actatattac ttgacaaaaa 540

ctttagtcag aaaaatatgg aatctctttc ggtactgcta agtgctaacc ttaaatagta 600

tagaattctt agttcattct caaaaacata gctatatgta gattataaaa gttcgatatt 660

atttcctgca aaagatgtta taatgttaca acttacaaga aaatgatgta tatgtagatt 720

ttataaactg gtaccgtaat tcataaaaga tggtggtggg tatgtatcag taacggaact 780

tacatatgcg tgtgtattac tatgtctata tggtgtattc ctttgtgtgg aacaatgcac 840

gtcagagttg tttattttct tatagaattt aaggaatcaa ttattggatt tctcaaggtg 900

aaagtggact tctttgcacg caaggtctag ttgccgactt gccgttgcat gtaacatgat 960

tgttgaaata aagtgaattg agagaagttt ggccagacat tttaaattta acccaaaaaa 1020

agtagggcct aacacaaaat ataacctctc tttgttcaaa ggaaataaca cctacgtctt 1080

ataattgaac caaacattga atcattgaac tcacctataa taattataat aacacgaatt 1140

cacaagacac ctaaaagaaa aagttcacaa aaacaaataa aaatttacct ctcaccaaac 1200

acactcacct acccgtctgg tcccactgac cccaacatac aacaccgact ctctcccaca 1260

ccaatttttt tttttggcgt tttaaaacaa ataaactatc tatttttttt tcttaccaac 1320

tgattaattc gtgaataatc tattatcttc ttcttttttt tgtgacggat gattagtgcg 1380

tggggaaatc aaaatttaca aaatttggga tgattccgat ttttgccatt cgattaattt 1440

tggttaaaag atatactatt cattcaccaa gttttcagat gagtctaaaa gataatatca 1500

tttcactagt cacttaaaaa aagggttaaa agaacatcaa taatatcact ggtttcctta 1560

ggtgacccaa aaaaagaaga aaaagtcact agtttctttt tggaaatttt actgggcata 1620

tagacgaagt tgtaatgagt gagtttaaat ttatctatgg cacgcagcta cgtctggtcg 1680

gactatacca agttaccaac tctctctact tcatgtgatt gccaataaaa ggtgacgtct 1740

ctctctctct caccaacccc aaaccacttt ccccactcgc tctcaaaacg cttgccaccc 1800

aaatctatgg cttacgggga catgtattaa catatatcac tgagtgaaaa gaagggttta 1860

ttaccgttgg accagtgatc aaacgtgttt tataaaaatt tggaattgaa aacatgattt 1920

gacattttta atgatggcag cagacgaaac caacaacact aagtttaacg ttcgtggagt 1980

atacttttct attttcgaag aagacatata actaagctga ttgttattct tcatagattt 2040

cttttcactg cgaataaaag tttgtgaaca tgtcaccgtt tgaacactca acaatcataa 2100

gcgttttacc tttgtggggt ggagaagatg acaatgagaa agtcgtcgta catataattt 2160

aagaaaatac tattctgact ctggaacgtg taaataatta tctaaacaga ttgcgaatgt 2220

tctctacttt ttttttgttt acattaaaaa tgcaaatttt ataacatttt acatcgcgta 2280

aatattcctg ttttatctat aattaatgaa agctactgaa aaaaaacatc caggtcaggt 2340

acatgtattt cacctcaact tagtaaataa ccagtaaaat ccaaagtaat taccttttct 2400

ctggaaattt tcctcagtag tttataccag tcaaattaaa acctcaaatc tgaatgttga 2460

aaatttgata tccaagaaat tttctcattg gaataaaagt tcaatctgaa aatagatatt 2520

tctctacctc tgtttttttt tttctccacc aactttcccc tacttatcac tatcaataat 2580

cgacattatc catctttttt attgtcttga actttgcaat ttaattgcat actagtttct 2640

tgttttacat aaaagaagtt tggtggtagc aaatatatat gtctgaaatt gattatttaa 2700

aaacaaaaaa agataaatcg gttcaccaac cccctcccta atataaatca aagtctccac 2760

cacatatatc tagaagaatt ctacaagtga attcgattta cacttttttt tgtccttttt 2820

tattaataaa tcactgaccc gaaaataaaa atagaagcaa aacttcatg 2869

<210> 31
<211> 1665
<212> DNA
<213> Arabidopsis thaliana

<400> 31
catttttagt tttctttcct ctgaaacgat tctccacgct ctttgtttat aaggattgca 60

agaaattact gaagaacatc aggaatcgcg agagcttggg gcgaatcaaa aacctccatt 120

gatgaagaag actgcatcta gtactttact tactcggtag cacacctcca tgggcaggct 180

ccagcaccat ggttgggccc atttcttatt aatgggccta aaatagagct tctagggcct 240

ctatatattt tgtcttttct cttccgttta ggcgttttgc taactttctt acgtatatga 300

atgtgaatta tagttagtta actttagaac tgttgagcgg ccaatgccac gtcctttgta 360

cgtaaattat gttattcatg ctattattta tgtaccaatt ttctattcta atatatattc 420

taagtttttt ttgtatcaat actaagtatt tatacaattt ttgcaaattt tgacagaaag 480

atagtacaaa aaatatgaaa gaccaaaaac tacataaacc accaaaaatt acaacacaaa 540

cagaaaacgg aaagaaaact caatcgaaaa ctctacaagt agagacctta aaagaaactt 600

agattccaaa ccacaaagct caaatatttg tgtggtttcc tcaacttaaa agatctcaaa 660

tacttatagt tgttgccact agcaatccaa acctaggcca gttttcaaaa tctctaaaaa 720

agagcttacc attccaaact taggacaatc ctcataatat tttaaatttt atgacatttt 780

ttagtttttt cttaccgaac atagagatac aacgaataac acaaaatacc aaattttact 840

aaacaaacaa ttactgcaca aatgaaaaag taatcaacaa catgagctca tttcacgaaa 900

aaagaagcga aaaagccaac ggtcttgcca caaattagta tattgcaaat tacaaatgct 960

taaaaaaatt cacagatata ttcatcaatt ataccttttg attatcttgc aaataaatct 1020

tccaattcat attacctacg tcaaaattta gccatctacc cttattatcc cattatgaaa 1080

atttcaaaat cgataagatg acagacaccc aacaacgaat tatcatttta tatactatag 1140

gtattagata cctacaattt atttggttta ttgggaatat acgattcata aattccctca 1200

taaaaatata aagcaaatgt ggacggaagc aaaaaaacta aatatagaat ggagagaaat 1260

tgtattatta acttaggtta cccatcgcca acaacctctc atttcctttc ctaaactatt 1320

tcacaatttt ggtcaaaaac acaatctaac cccaaaaaag attcgacctt tgaaattccc 1380

aaaaccagaa gcaaaacccc caaaagaacc ctacttatag tgaaagaaaa gtaacataat 1440

aatatatata cacgagaacc aagagccact tgaatataaa gttcacagtt tttgtctgct 1500

caccaaatca aaccaatttc tcctgatagc caaagttttt gtccccaaca agaaactcat 1560

gcaacattct tcacccacta gctaactaac gacttccatt cttattaatc tctctcaatt 1620

cttcatttct gagattcatc gctctctctt ctctttgtca caatg 1665

<210> 32
<211> 2088
<212> DNA
<213> Arabidopsis thaliana

<400> 32
taacatctac aaagcctata aaatggaaag atgtatgcat cgatgatgat tctgcctcac 60

ttagaagaat caaaaagcta aaaggagctg cgaatcctgg gcaacgatga cttagtatgt 120

atagaagaaa aaaaaagctt ttacttggta tcaaacagag catgtgttgt tgtatttgct 180

acctttttct gcaaagttat tggcaattgt attgttgttc cacttgtata tatacagaga 240

ccatttatgt taatatacaa cttcgtatgg ggaaagatga ttccatcaag tccaaaatgc 300

caaaaccatg cacgatcttg tctgatcaag tatgataaga tgtgagattt taagaacgtg 360

aagcttttca tattgtattc tcaatgaacg aagaattaat aattagagtt agaaatgcag 420

attacagaga ttacacaatt tgatctcatt tcttggcaaa gaggcccagg ccaccgaagc 480

caccgtcctt ctttggattc ctgtgtgtta attttcgtgt ggctttagtt acaaagggat 540

tatattgtca gcgaatctga gccgttggat cgttttgaca tgagctttcc ataagtgtct 600

tatcctttgt ttctctcatt tttcacatat gtatcagaca tttaatcaag cacatatcta 660

cgtgttgatt atgttgatga tgctgagatt atgttgattt agagaatgag tattggtgat 720

gatggtacac gttagtaaac atatgaattt ggaagtgcca cgtgagaggg agcagagttt 780

tgggctttct tttggttatg tatttgcatt cttgttgtaa caaattctct tattaaaagc 840

attgtgaaaa aaaccaaata ccacatagtc tatgtttcaa gatttgtttt ttaatactat 900

aattttctat tactttagtt tgtgtaattt ttgaaaagag aaaattggta ttttttagga 960

ttgggtgatc tgaaatttga ctacaaaaat tttcttggac acagacacac agtcacacaa 1020

ataagctaat tattggaatt ttatcttaag ttgcattcac tttgcaaatt aatattgaat 1080

tatagactat tagtgtatgg cgtgatggca aacaaaaaaa tcagtgtgtg atattgtgga 1140

ctagccaaaa aaattttctg atgatttttt tgcctaaata atttgaattg ttgagaccga 1200

aactcttact aatatactaa caatgtaacg cgacaaataa aacattgttg agaataaaat 1260

ctgaaccatc ataactatag tctatagtaa ccgtccgaat tcaaccacat atgtatgatc 1320

tttgccattt ttgatcatat tttccgttgc gttttctttg aattttctca atatattttt 1380

agtgttttga tttgtacttt tatctaattt atataattgt ttttcaaatg tttcgcaatt 1440

gtgcgtttaa gtattcgact cgaagtaaac ccagtttctt agaatgggct aactgccatt 1500

gaccaaatct ttatccgcga agaaagatct ttagcagcta ggcttatttc atatcaacaa 1560

aagtatatat ttatctctaa ctaaattcct acaattattt caaaaacata gaaattaaaa 1620

tttgaaatgc ctattctttt ttggttatac attgcaaagg ttatgtatgt aatttctgat 1680

cttgaaattt aagttcttta gcccatatct agttgatttt cacgaaatat tgtcgtttac 1740

tctcgttttt atatataaag catgtacata taacatataa tcaatgcttc aaaagaaaaa 1800

aataatcata actaaattga aacgggaaaa ctaatttgct ttcgtgttct attatgccaa 1860

actgtactca caatagtctt atcctatcga cccaaattta atcttcccaa ctacttactc 1920

ctaattgaaa caaaatataa cctttcttac tatagcctat atttaaaaaa tatctatgat 1980

ttctacattt atatatatac gcacacggca catacctctc attcaccata tatgcctctt 2040

aacaatctct ccacagaaca aaagcaaaaa gcctaaaccg ggataatg 2088

<210> 33
<211> 2897
<212> DNA
<213> Arabidopsis thaliana

<400> 33
gaaaatcagc gtaatgagag acgagagcaa tgtgctaaga gaagagattg ggaagagaga 60

agagacgata aaggaaacgg aaaagcatat ggaggagctt catatggagc aagtgaggct 120

gagaagacgg tcgagtgagc ttacggaaga agtggaaagg acgagagtgt ctgcatcgga 180

aatggctgag cagaaaagag aagctataag acagctttgt atgtctcttg accattacag 240

agatgggtac gacaggcttt ggagagttgt tgccggccat aagagtaaga gagtagtggt 300

tttaacaact tgaagtgtaa gaacaatgag tcaatgacta cgtgcaggac attggacata 360

ccgtgtgttc ttttggattg aaatgttgtt tcgaagggct gttagttgat gttgaaaata 420

ggttgaagtt gaataatgca tgttgatata gtaaatatca atggtaatat tttctcattt 480

cccaaaactc aaatgatatc atttaattat aaactaacgt aaactgttga caatacactt 540

atggttaaaa atttggagtc ttgttttagt atacgtatca ccaccgcacg gtttcaaaac 600

cacataattg taaatgttat tggaaaaaag aacccgcaat acgtattgta ttttggtaaa 660

catagctcta agcctctaat atataagctc tcaacaattc tggctaatgg tcccaagtaa 720

gaaaagccca tgtattgtaa ggtcatgatc tcaaaaacga gggtgaggtg gaatactaac 780

atgaggagaa agtaaggtga caaatttttg gggcaatagt ggtggatatg gtggggaggt 840

aggtagcatc atttctccaa gtcgctgtct ttcgtggtaa tggtaggtgt gtctctcttt 900

atattattta ttactactca ttgttaattt ctttttttct acaatttgtt tcttactcca 960

aaatacgtca caaatataat actaggcaaa taattattta attgtaagtc aatagagtgg 1020

ttgttgtaaa attgattttt gatattgaaa gagttcatgg acggatgtgt atgcgccaaa 1080

tgctaagccc ttgtagtctt gtactgtgcc gcgcgtatat tttaaccacc actagttgtt 1140

tctctttttc aaaaacacac aaaaaataat ttgttttcgt aacggcgtca aatctgacgg 1200

cgtctcaata cgttcaattt tttctttctt tcacatggtt tctcatagct ttgcattgac 1260

cataggtaaa gggataagga taaaggtttt ttctcttgtt tgttttatcc ttattattca 1320

aaatggataa aaaaacagtc ttattttgat ttctttgatt aaaaaagtca ttgaaattca 1380

tatttgattt tttgctaaat gtcaactcag agacacaaac gtaatgcact gtcgccaata 1440

ttcatggatc atgaccatga atatcactag aataattgaa aatcagtaaa atgcaaacaa 1500

agcattttct aattaaaaca gtcttctaca ttcacttaat tggaatttcc tttatcaaac 1560

ccaaagtcca aaacaatcgg caatgttttg caaaatgttc aaaactattg gcgggttggt 1620

ctatccgaat tgaagatctt ttctccatat gatagaccaa cgaaattcgg catacgtgtt 1680

tttttttttg ttttgaaaac cctttaaaca accttaattc aaaatactaa tgtaacttta 1740

ttgaacgtgc atctaaaaat tttgaacttt gcttttgaga aataatcaat gtaccaataa 1800

agaagatgta gtacatacat tataattaaa tacaaaaaag gaatcaccat atagtacatg 1860

gtagacaatg aaaaacttta aaacatatac aatcaataat actctttgtg cataactttt 1920

tttgtcgtct cgagtttata tttgagtact tatacaaact attagattac aaactgtgct 1980

cagatacatt aagttaatct tatatacaag agcactcgag tgttgtcctt aagttaatct 2040

taagatatct tgaggtaaat agaaatagtt aactcgtttt tattttcttt tttttaccat 2100

gagcaaaaaa agatgaagta agttcaaaac gtgacgaatc tacatgttac tacttagtat 2160

gtgtcaatca ttaaatcggg aaaacttcat catttcagga gtactacaaa actcctaaga 2220

gtgagaacga ctacatagta catattttga taaaagactt gaaaacttgc taaaacgaat 2280

ttgcgaaaat ataatcatac aagtagaacc actgatttga tcgaattatt catagctttg 2340

taggatgaac ttaactaaat aatatctcac aaaagtattg acagtaacct agtactatac 2400

tatctatgtt agaatatgat tatgatataa tttatcccct cacttattca tatgattttt 2460

gaagcaacta ctttcgtttt tttaacattt tcttttttgg tttttgttaa tgaacatatt 2520

tagtcgtttc ttaattccac tcaaatagaa aatacaaaga gaactttatt taatagatat 2580

gaacataatc tcacatcctc ctcctacctt caccaaacac ttttacatac actttgtggt 2640

ctttctttac ctaccaccat caacaacaac accaagcccc actcacacac acgcaatcac 2700

gttaaatcta acgccgttta ttatctcatc attcaccaac tcccacgtac ctaacgccgt 2760

ttaccttttg ccgttggtcc tcatttctca aaccaaccaa acctctccct cttataaaat 2820

cctctctccc ttctttattt cttcctcagc agcttcttct gctttcaatt actctcgccg 2880

acgattttct caccgga 2897